# FASCICULE DE BREVET EUROPEEN

(11) **EP 1 417 349 B1**
(45) Date de publication et mention de la délivrance du brevet: **19.11.2008**
(21) Numéro de dépôt: 02777405.8
(22) Date de dépôt: 13.08.2002
(51) Int. Cl.: C12Q 1/68, C12N 9/16

(54) **NOUVELLE CIBLE MOLECULAIRE DE LA NEUROTOXICITE**
NEUES MOLEKULARES ZIEL FÜR NEUROTOXIZITÄT
NOVEL MOLECULAR TARGET FOR NEUROTOXICITY

(30) Priorité: 14.08.2001 FR 0110819
(43) Date de publication de la demande: 12.05.2004
(60) Demande divisionnaire: 08160783.0
(73) Titulaire: Exonhit Therapeutics S.A., 75017 Paris (FR)
(72) Inventeur: AÏT IKHLEF, Ali, 94140 Alfortville (FR); RESINK, Annelies, 3001 Heverlee (BE); SCHWEIGHOFFER, Fabien, F-94130 Nogent sur Marne (FR)
(74) Mandataire: Tezier Herman, Béatrice
(86) Numéro de dépôt international: PCT/FR2002/002861
(87) Numéro de publication internationale: WO 2003/016563

(56) Documents cités:
- WO-A-00/40714
- WO-A-01/44449
- US-A- 5 851 784
- US-A- 5 977 305
- US-A- 6 060 501
- CHERRY J A ET AL: "Diazepam and rolipram differentially inhibit cyclic AMP-specific phosphodiesterases PDE4A1 and PDE4B3 in the mouse" BIOCHIMICA ET BIOPHYSICA ACTA. GENE STRUCTURE AND EXPRESSION, ELSEVIER, AMSTERDAM, NL, vol. 1518, no. 1-2, 19 mars 2001 (2001-03-19), pages 27-35, XP004275852 ISSN: 0167-4781 & DATABASE EMBL ebi; 23 décembre 1999 (1999-12-23), XP002248838
- MISHRA S K ET AL: "CALCIUM CALMODULIN AND 3' 5' CYCLIC NUCLEOTIDE PHOSPHODIESTERASE ACTIVITY IN HUMAN MUSCULAR DISORDERS" JOURNAL OF THE NEUROLOGICAL SCIENCES, vol. 109, no. 2, juin 1992 (1992-06), pages 215-218, XP001064869 ISSN: 0022-510X
- PRICE D L ET AL: "AMYOTROPHIC LATERAL SCLEROSIS AND ALZHEIMER'S DISEASE LESSONS FROM MODEL SYSTEMS" REVUE NEUROLOGIQUE, MASSON, PARIS, FR, vol. 153, no. 8/9, septembre 1997 (1997-09), pages 484-495, XP000982876 ISSN: 0035-3787

## Description

La présente invention concerne le domaine de la biologie, de la génétique et de la médecine. Elle concerne notamment de nouvelles méthodes pour le traitement (ou la prise en charge) de pathologies neurodégénératives, et en particulier de la sclérose latérale amyotrophique. L'invention découle notamment de l'identification du rôle de la phosphodiestérase 4B dans ces pathologies et décrit son utilisation comme cible ou marqueur thérapeutique, diagnostique ou expérimental de ces désordres.

De nombreuses pathologies neurodégénératives ont été décrites comme ayant une composante ou un stade lié au phénomène d'excitotoxicité. C'est le cas de la maladie d'Alzheimer, de la maladie de Parkinson, de la sclérose en plaques et de la chorée de Huntington. Pour la sclérose en plaques, il est notamment décrit dans le brevet US 6060501 un traitement préventif ou curatif en administrant une combinaison d'inhibiteurs de PDE4 et d'agents antiinflammatoires ou immunomodulateurs.

La sclérose amyotrophique latérale (SAL ou ALS pour Amyotrophic Lateral Sclerosis) est une maladie neurodégénérative associée à différents types d'inclusions tels les corps de Lewis et caractérisée par une apoptose des motoneurones spinaux et corticaux dont l'issue fatale est parfois associée à une démence frontale. Des formes sporadiques, sans aucune mutation décrite, coexistent avec des formes familiales (FALS) associées à des mutations dans le gène SOD1 codant pour la superoxide dismutase. La majorité des cas est sporadique, les formes familiales (FALS) étant très rares. Il est vraisemblable qu'une longue période asymptomatique précède l'apparition des symptômes cliniques qui sont variés et dont la classification est complexe. Les futurs développements thérapeutiques substitueront aux traitements de la symptomatologie des stratégies basées sur les causes moléculaires de la pathologie. Au niveau cellulaire, ces symptômes sont associés à une mort des motoneurones corticaux et des motoneurones spinaux. Cette mort neuronale a été reliée à différents phénomènes qui constituent la base de plusieurs pathologies neurodégénératives. C'est le cas de l'excitotoxicité liée au glutamate, du stress oxydatif, d'une certaine auto immunité dirigée contre des marqueurs neuronaux (les canaux calciques dans le cas de l'ALS) ainsi que d'anomalies du cytosquelette. Si ces phénomènes sont décrits, la ou les causes de ces maladies, dont l'ALS, sont obscures. Même si les FALS sont liées à des mutations dans le gène SOD1 qui code pour la superoxide dismutase, les mécanismes qui engagent les neurones vers la mort cellulaire dont au moins une composante est l'apoptose, sont inconnus.

L'identification des évènements moléculaires impliqués dans les différents phénomènes impliqués dans la mort cellulaire permettra de mettre en place de nouvelles stratégies thérapeutiques. L'étude de ces évènements est difficilement réalisable à partir de biopsies humaines. Ces biopsies proviennent évidemment d'échantillons post-mortem dont la qualité est difficilement contrôlable et ne représentent que des états pathologiques représentatifs des phases tardives de la maladie.

Les modèles animaux donnent accès à des échantillons biologiques qui permettent d'analyser différentes étapes du développement d'une pathologie et de comparer ces étapes à des témoins sains. A cet égard, des souris transgéniques qui expriment le gène humain SOD1 portant l'une des mutations qui prévaut dans les FALS (mutation G93A) sont disponibles auprès de Jackson Laboratory, sous condition de prise d'une licence d'utilisation auprès de la NorthWestern University. Ce modèle reproduit en 120 jours l'issue fatale de la maladie avec des symptômes comparables à ceux de la maladie humaine. L'apparition des symptômes d'ALS liés à la mutation G93A dans SOD1 n'est pas la conséquence d'une réduction de l'activité superoxyde dismutase mais d'un gain de fonction qui augmente la capacité de l'enzyme à générer des radicaux libres. Malgré ces informations, les évènements moléculaires qui président aux différentes étapes de l'ALS sont mal connus. La complexité de ces évènements moléculaires reflète l'évolution de la pathologie : Dans le modèle transgénique étudié, aucune dérégulation neuronale ou manifestation clinique n'a été rapportée à 30 jours. 60 jours correspondent à un stade qui précède de peu les premiers symptômes, mais qui est déjà caractérisé au niveau cérébral par des changements dans la physiologie cellulaire tels qu'une altération du métabolisme mitochondrial, un stress et une mort neuronale associés à un phénomène d'excitotoxicité. A 90 jours, 50% des motoneurones corticaux et spinaux sont morts et un processus actif d'apoptose neuronale est engagé parallèlement à une activation astrocytaire. Le phénomène d'excitotoxicité n'est plus observé à ce stade. La mort neuronale y est associée à l'activation de caspases qui ne semblent pas impliquées dans les phases précoces de la pathologie.

Identifier les différents évènements moléculaires spécifiques des différentes phases de la pathologie doit permettre d'identifier de nouvelles cibles thérapeutiques aussi bien que de nouveaux marqueurs diagnostiques. L'une des approches les plus efficaces pour réaliser cette identification consiste à identifier les gènes et les protéines dont l'expression caractérise un état physiopathologique.

La présente invention décrit à présent l'identification d'événements génétiques impliqués dans les phénomènes d'excitotoxicité et de mort neuronale. La présente invention fournit ainsi de nouvelles approches thérapeutiques et diagnostiques des pathologies associées à ces phénomènes, ainsi que de nouvelles cibles pour l'identification de composés actifs.

Plus particulièrement, une analyse qualitative différentielle a été effectuée à partir d'ARN extraits d'échantillons de cerveau et de moelle épinière, sans isolement préalable des neurones afin de prendre en compte un maximum d'évènements d'épissages alternatifs liés au développement de la pathologie. Cette analyse a été effectuée par criblage différentiel qualitatif selon la technique DATAS (décrite dans la demande n° WO99/46403), qui présente des avantages inégalés.

La présente demande de brevet découle notamment de la construction par la demanderesse d'un répertoire des altérations d'épissage dans le cerveau des animaux modèles de l'ALS âgés de 60 jours. Ce répertoire, qui contient plus de 200 séquences distinctes, implique des acteurs clefs du phénomène d'excitotoxicité tels que les canaux potassiques et le récepteur NMDA. Des séquences dérivées d'ARNs codant pour des protéines impliquées dans la réponse au stress, dont des protéines de choc thermique, font également partie de ce répertoire, soulignant l'implication de cette réponse dans les phases précoces de l'ALS. Une altération du métabolisme énergétique apparaît clairement affecter les motoneurones corticaux des animaux qui développent la pathologie. Par exemple, l'intron 6 de la forme mitochondriale de la créatine kinase est isolé spécifiquement à partir des ARN messagers exprimés en conditions pathologiques chez les animaux âgés de 60 jours. Cette interruption de la séquence codante par cette rétention d'intron aboutit à un ARN messager qui code pour une forme inactive de l'enzyme. Cette observation est en accord avec les observations biochimiques qui ont montré une diminution de l'activité créatine kinase mitochondriale corrélée avec une diminution de la quantité de cette enzyme dans les neurones des animaux du même modèle transgénique. La spécificité des séquences qui constituent ce répertoire est attestée par le fait que la même analyse différentielle qualitative de l'expression génétique réalisée sur des animaux âgés de 90 jours aboutit à un répertoire différent dont sont absents notamment les différents marqueurs de l'excitotoxicité. L'analyse des modifications d'épissage confirme que les évènements moléculaires sont différents selon le stade de la pathologie.

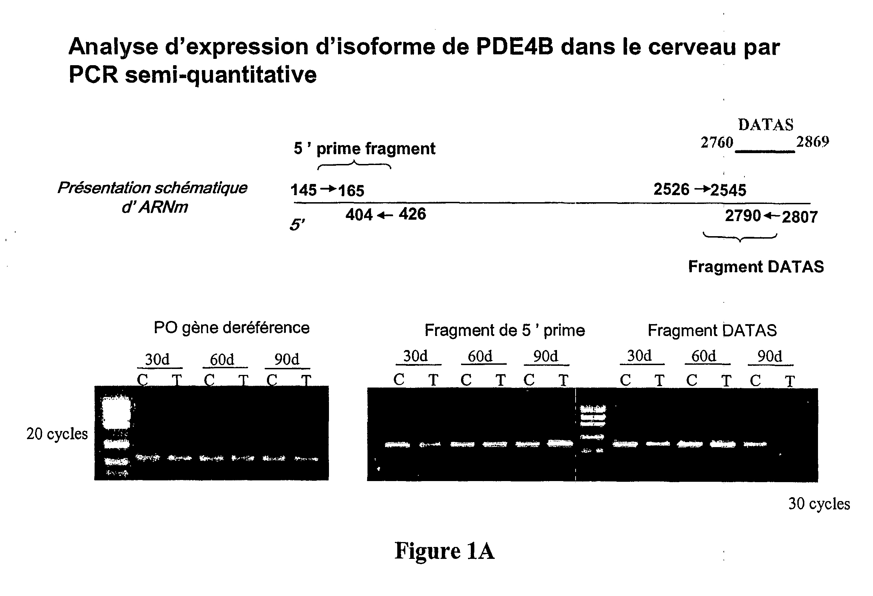

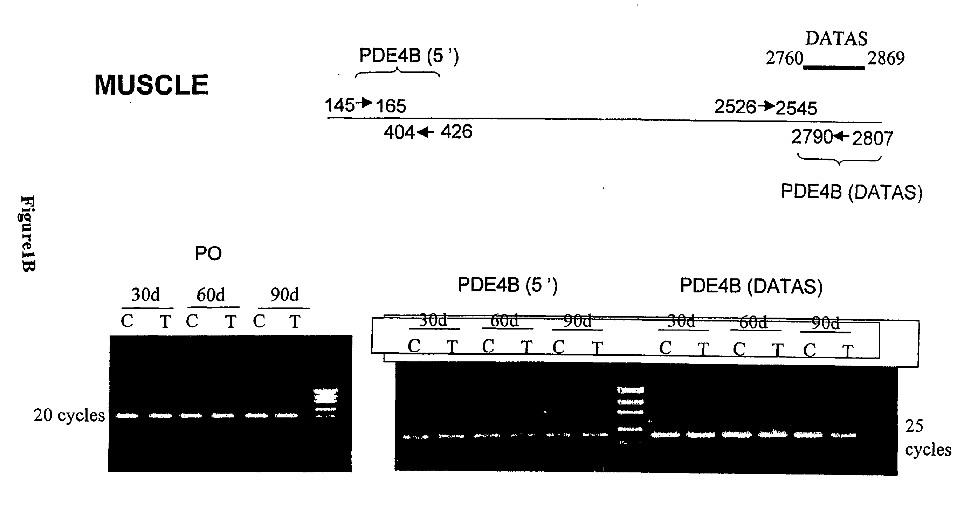

De manière particulièrement intéressante et inattendue, la réalisation de DATAS sur des ARN d'animaux contrôles et transgéniques âgés de 60 jours a permis d'isoler un fragment d'ADNc dérivé de l'ARNm de la phosphodiestérase 4B. Ce fragment correspond à un fragment d'exon spécifiquement présent dans les animaux contrôles et donc spécifiquement délété dans les animaux transgéniques pour SOD1G93A au stade 60 jours. Ce fragment recouvre les nucléotides 377 à 486 référencés à partir du codon stop de la PDE4B de souris (SEQ ID NO :1) (séquence également accessible dans GenBank, n°AF208023). Cette séquence comprend 2912 bases, le fragment délété correspondant aux bases 2760 à 2869. Cette région est non codante et est exprimée différentiellement entre les animaux contrôles et les animaux transgéniques, du fait de l'utilisation alternative d'un exon 3' non codant ou du fait de l'utilisation de deux sites de polyadénylation alternatifs. Cette expression différentielle a été mise en évidence par des expériences de RT PCR présentées sur les figures 1A et 1B.

La présente demande démontre donc l'implication de la phosphodiestérase 4B dans le développement des processus d'excitotoxicité et de mort neuronale. Les résultats obtenus montrent une expression plus prononcée de PDE4B dans les tissus nerveux pathologiques, liée à une modification structurale de l'ARN correspondant, notamment à la délétion d'une région dans la partie 3' non-codante. Ce résultat est tout à fait compatible avec la présence de séquences de déstabilisation des ARNm dans la séquence identifiée par DATAS. Leur délétion de l'ARNm de la PDE4B, par épissage ou par utilisation de séquences de polyadénylation alternatives, peut aboutir à une stabilisation, donc à une augmentation de l'expression de la partie codante de cet ARN. Cet événement se produit spécifiquement dans le cerveau des sujets pathologiques et non dans les sujets contrôles.
La présente invention décrit donc un événement moléculaire original qui aboutit à une augmentation de l'expression de l'ARNm de la PDE4B dans le cerveau des sujets pathologiques et qui est corrélé dans le temps avec le phénomène d'excitotoxicité et/ou de mort neuronale. L'invention montre également, pour la première fois, qu'une augmentation de l'expression de la PDE4B est associée aux stades précoces de l'ALS. La PDE4B constitue donc une cible thérapeutique nouvelle et importante dans le développement de thérapeutiques de ces pathologies, utilisables notamment à des phases précoces de leur développement, et s'adressant aux véritables bases moléculaires de la pathologie et non aux symptômes ou composantes inflammatoires associées.

L'invention décrit également de nouvelles méthodes de diagnostic, dépistage, détection, détermination d'une prédisposition ou de suivi de l'évolution ou de l'efficacité du traitement de ces pathologies.

### Détection, Diagnostic et dépistage

La présente invention décrit donc une méthode de détection d'une situation d'excitotoxicité ou de stress neuronal chez un sujet, comprenant la mesure in vitro de l'expression de la phosphodiestérase 4, notamment de la phosphodiestérase 4B dans un échantillon provenant du sujet. La méthode comprend avantageusement une mesure de l'expression différentielle de la région 3' non-codante du gène PDE4B et du reste du gène, notamment de la partie codante.

L'invention décrit donc une méthode de détection d'une situation d'excitotoxicité ou de stress neuronal chez un sujet, comprenant la détection de la présence d'une forme mutée de l'ARN de la phosphodiestérase 4, notamment de la phosphodiestérase 4B dans un échantillon provenant du sujet, en particulier d'une forme délétée de tout ou partie de la région 3' non-codante.

L'invention divulgue également l'utilisation d'un acide nucléique comprenant tout ou partie d'une séquence dérivée du gène ou de l'ARN messager de la PDE4B pour la mise en oeuvre d'une méthode de diagnostic ou de détection d'une situation de stress neuronal et plus particulièrement la situation d'excitotoxicité.

L'invention réside, généralement, dans l'utilisation d'un acide nucléique complémentaire de tout ou partie du gène ou du messager de la PDE4B, pour la détection d'événements pathologiques de type excitotoxicité, stress ou mort neuronale, etc. Plus généralement, l'invention repose sur une méthode de diagnostic, dépistage, caractérisation ou suivi d'une pathologie dégénérative, comprenant la mise en évidence d'une altération dans le gène PDE4 ou dans l'ARN correspondant, typiquement PDE4B.

L'expression de la PDE4, ou le différentiel d'expression, ou la présence d'une forme altérée peuvent être déterminés par des techniques conventionnelles de biologie moléculaire, comme par exemple par séquençage, hybridation, amplification, RT-PCR, migration sur gel, etc. L'invention est applicable au diagnostic ou la détection de différentes pathologies impliquant les phénomènes d'excitotoxicité, telles que la maladie d'Alzheimer, la maladie de Parkinson, la sclérose en plaques, l'ALS, la chorée de Huntington ou l'ischémie cérébrale. Elle peut être utilisée pour la détection précoce, la mise en évidence d'une prédisposition, le choix et l'adaptation d'un traitement, le suivi de l'évolution de la pathologie, etc. Elle est particulièrement adaptée à la détection à un stade précoce de la sclérose en plaques ou de l'ALS.

Pour la mise en oeuvre des méthodes génétiques de diagnostic ou de détection selon l'invention, on utilise plus particulièrement des acides nucléiques capables de mettre en évidence une forme délétée de l'ARNm de la PDE4B, notamment une forme dépourvue de tout ou partie de la région 3' non codante. A titre d'exemple spécifique, on utilise un acide nucléique complémentaire de tout ou partie de la région comprise entre les résidus 2760 à 2869 de la séquence SEQ ID NO :1, ou des résidus correspondants de la séquence du gène ou de l'ARNm de la PDE4B humaine. La séquence de l'ADNc codant la PDE4B humaine et de la protéine correspondante sont représentées dans les séquences SEQ ID NO : 3 et 4 (voir également Genbank, n° NM_002600). La région 3' non-codante de l'ARN ou du gène PDE4B humain correspond aux résidus 2461 à 4068 de SEQ ID NO :3.

Avantageusement, l'acide nucléique utilisé (comme sonde) comprend tout ou partie de la séquence codant la région 3' non-codante du gène ou de l'ARN de la PDE4B comprise entre les nucléotides 2384 et 2869 de la séquence SEQ ID NO :1 ou entre les nucléotides 2461 et 4068 de la séquence SEQ ID NO :3 ou une séquence complémentaire de celles-ci.

Selon des modes particuliers de mise en oeuvre, l'invention utilise un acide nucléique complémentaire d'une région comprise dans une séquence suivante :
- résidus 2384 à 2869 de SEQ ID n° 1
- résidus 2500 à 2869 de SEQ ID n° 1
- résidus 2760 à 2869 de SEQ ID n° 1
- résidus 2780 à 2850 de SEQ ID n° 1
- résidus 2790 à 2810 de SEQ ID n° 1
- résidus 2600 à 4040 de SEQ ID n° 3
- résidus 3000 à 4040 de SEQ ID n° 3
- résidus 3500 à 4040 de SEQ ID n° 3
- résidus 3900 à 4040 de SEQ ID n° 3.

Selon un autre mode particulier, on utilise un acide nucléique complémentaire de la séquence de la région de l'ARN de PDE4 résultant de la délétion de tout ou partie de la partie 3' non codante. L'élimination d'un domaine créé en effet de nouvelles jonctions dans la séquence, qui sont spécifiques de la forme délétée et peuvent être utilisées pour mettre en évidence la présence d'une telle forme dans un échantillon.

La complémentarité entre la sonde et la séquence cible est, de préférence, parfaite pour assurer une meilleure spécificité d'hybridation. Toutefois, il est entendu que certains mésappariements peuvent être tolérés. L'acide nucléique utilisé pour la mise en oeuvre des méthodes ci-dessus peut être un ADN ou un ARN, de préférence un ADN d'origine synthétique. Il comporte de préférence de 10 à 500 bases, typiquement de 10 à 100 bases. Il est entendu qu'un acide nucléique plus long peut être utilisé, si désiré, bien que cela ne soit pas préféré. L'acide nucléique est avantageusement un ADN simple brin, de 10 à 500 bases, complémentaire d'une région au moins de la séquence 3'-non codante de la PDE4B. L'acide nucléique peut être marqué, par exemple par voie radioactive, enzymatique, luminescente, fluorescente, chimique, etc.

Une autre approche pour détecter la présence d'une altération du gène PDE4 utilise une amorce ou un couple d'amorces nucléiques permettant une amplification sélective d'une portion de l'ARN PDE4, de préférence comprenant une portion de la région 3' non codante. On utilise typiquement une amorce permettant l'amplification sélective de la forme altérée de l'ARN de PDE4, notamment d'une amorce spécifique de la jonction créée par l'élimination du partie de la région 3' de l'ARN.

A cet égard, l'invention décrit dans une amorce complémentaire d'une partie de la région 3' non-codante de la PDE4B, et permettant l'amplification d'une partie de cette région. L'amorce comporte avantageusement de 8 à 20 bases. Elle est préférentiellement composée d'un fragment de 8 à 20 résidus consécutifs de la séquence comprise entre les nucléotides 2384 et 2869 de la séquence SEQ ID NO :1 ou entre les nucléotides 2461 et 4068 de la séquence SEQ ID NO :3 ou d'une séquence complémentaire de celles-ci. L'invention décrit un couple d'amorce permettant l'amplification spécifique d'une partie au moins de la région 3' non-codante de la PDE4, ledit couple comprenant au moins une amorce telle que définie ci-dessus.

Pour la mise en oeuvre des méthodes décrites, on met en contact in vitro un échantillon biologique d'un sujet, contenant un acide nucléique, avec un acide nucléique (sonde, amorce, etc.) tel que défini ci-dessus, et on détecte la formation d'un hybride ou d'un produit d'amplification. L'échantillon biologique peut être un échantillon de sang, de fluide, de cellule, de tissu, etc. L'acide nucléique peut être immobilisé sur un support, de type verre, silice, nylon, etc.

Le procédé de détection, dépistage ou diagnostic peut être mis en oeuvre à partir de différents types d'échantillons provenant d'un sujet, comme par exemple des biopsies de tissus, notamment de tissu nerveux. De manière particulièrement surprenante et avantageuse, la présente invention montre par ailleurs que la dérégulation de l'expression de PDE4, corrélée au phénomène d'excitotoxicité, peut être mise en évidence directement dans le tissu musculaire. Ceci est tout particulièrement remarquable dans le cas de pathologies neurodégénératives telles que l'ALS.

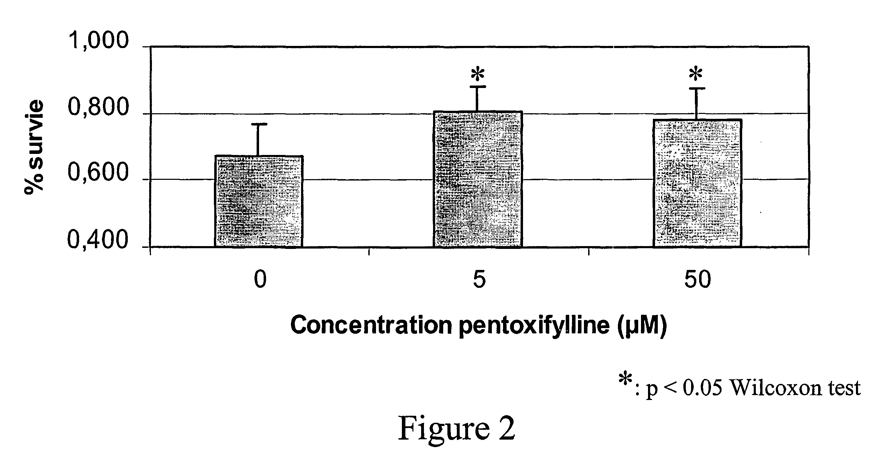

Au cours du développement de l'ALS, les phénomènes dégénératifs se produisent non seulement dans le cerveau mais également dans la moelle épinière et en conséquence dans le muscle par défaut d'innervation. La figure 2 présente les modifications d'expression de l'ARNm de la PDE4B dans les muscles de souris contrôles et transgéniques, suivies en utilisant les mêmes amorces de PCR que lors de l'étude sur les ARN de cerveaux de ces mêmes animaux. De façon analogue, toutefois moins prononcée, une diminution de l'expression de la région 3' non codante de la PDE4B, et non du reste de cet ARNm (notamment la partie codante), est observée spécifiquement dans le muscle des animaux en fin de phase pré-symptomatique, c'est à dire âgés de 90 jours.
L'une des difficultés rencontrées lors des études et des traitements de l'ALS est la difficulté de poser un diagnostic précoce. Cette observation de dérégulation de l'ARNm de la PDE4B dans le muscle ALS permet de proposer un diagnostic précoce à partir de biopsies musculaires de patients. Ce diagnostic est basé sur la détection de l'expression différentielle de la région 3' non codante et du reste de la séquence, notamment codante, de la PDE4B.

Un procédé particulier de détection d'une situation de stress neuronal, notamment d'excitotoxicité, en particulier liée à une pathologie neurodégénérative chez un sujet, comprend la mesure de l'expression du gène PDE4B, ou de la présence de formes délétées du messager de PDE4B, dans un échantillon de cellules musculaires provenant dudit sujet.

Pour mesurer l'expression différentielle, on utilise par exemple une sonde correspondant à (c'est-à-dire spécifique de) une partie de la région 3' non codante et une sonde correspondant à une partie de la région codante de la PDE4B. Le signal détecté avec chacune de ces sondes permet d'évaluer le différentiel d'expression. Une autre approche utilise deux couples d'amorces permettant une amplification d'une portion de la région 3' non codante d'une part et d'une partie de la région codante d'autre part.

La présente invention divulgue également un kit pour l'analyse de l'expression de la PDE4, notamment de l'expression différentielle entre la région 3' non-codante et la région codante, le kit comprenant une sonde nucléotidique spécifique d'une partie de la séquence de la région 3' non-codante et une sonde nucléotidique spécifique d'une partie de la séquence de la région codante.

La présente invention divulgue également un kit pour l'analyse de l'expression de la PDE4, notamment de l'expression différentielle entre la région 3' non-codante et la région codante, le kit comprenant un couple d'amorces nucléotidiques permettant l'amplification spécifique d'une partie au moins de la région 3' non-codante de la PDE4 et un couple d'amorces nucléotidiques permettant l'amplification spécifique d'une partie au moins de la région codante de la PDE4.

### Thérapie

Les phosphodiestérases hydrolysent les acides nucléiques cycliques tels l'AMPc et le GMPc, régulant différentes cascades de signalisation. La PDE4B hydrolyse l'AMPc, régulant ainsi la concentration intracellulaire de ce second messager. L'implication de l'AMPc dans la balance qui existe entre la viabilité cellulaire et l'apoptose est bien décrite dans la littérature. Notamment, la cascade AMPc est bien intégrée dans les cascades de survie cellulaire impliquant des kinases telles Akt et P13K, de même que dans la régulation de l'activité du facteur de transcription CREB. Il est à noter que ce facteur de transcription est impliqué dans la survie neuronale et la croissance des neurites. Toutefois, l'utilisation d'inhibiteurs de PDE et avantageusement de PDE4 n'a jamais été envisagée pour améliorer la viabilité neuronale et plus particulièrement leur protection contre l'excitotoxicité. Les inhibiteurs de PDE4, développés pour inhiber les phénomènes inflammatoires, ont été suggérés comme potentiellement utiles dans des pathologies neurodégénératives comme la maladie d'Alzheimer. Cette suggestion s'appuie sur la volonté de réduire les inflammations qui sont observées dans le cerveau au cours des processus neurodégénératifs et nullement sur un rationnel visant à inhiber directement la mort neuronale.

La présente invention montre l'existence d'événements d'épissage ou de sites de polyadénylation alternatifs affectant le gène de la PDE4, associés au développement de l'excitotoxicité neuronale, et fournit la base moléculaire qui justifie l'utilisation d'inhibiteurs de PDE4 pour le traitement de l'ALS et plus généralement pour améliorer la viabilité neuronale lors des phénomènes d'excitotoxicité, en particulier dès les phases précoces de ces pathologies.

Un objet de l'invention réside donc dans l'utilisation d'un composé capable d'inhiber ou de réduire l'expression ou l'activité de la PDE4B, pour la préparation d'une composition destinée au traitement de l'ALS, notamment en phase précoce, plus préférentiellement pour réduire l'excitotoxicité neuronale précoce associée à l'ALS.

Un objet particulier réside dans l'utilisation d'un inhibiteur de PDE4B pour la préparation d'une composition destinée au traitement de l'ALS, notamment pour réduire l'excitotoxicité chez les sujets atteints d'ALS ou pour augmenter la survie neuronale chez les sujets atteints d'ALS.

Un autre objet de l'invention réside dans l'utilisation d'un composé capable d'inhiber (de préférence de manière sélective) l'expression ou l'activité de la PDE4B de séquence SEQ ID n° 2 ou 4 pour la préparation d'une composition destinée à réduire l'excitotoxicité neuronale.

Un autre objet de l'invention réside dans une méthode de traitement d'une pathologie associée à un stress neuronal, notamment à une excitotoxicité, comprenant l'administration à un sujet d'un composé inhibiteur de l'activité ou de l'expression de la PDE4B, de préférence un composé inhibiteur sélectif de PDE4.

Un autre objet de l'invention réside dans une méthode de traitement de l'ALS, notamment dans une méthode pour augmenter la survie des neurones chez des patients atteints d'ALS, comprenant l'administration à un sujet d'un inhibiteur de PDE4B.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie), etc. Le traitement peut en outre être réalisé en combinaison avec d'autres agents ou traitements, notamment adressant les événements tardifs de la pathologie, tels que des inhibiteurs de caspases ou autres composés actifs.

On utilise dans la présente invention comme inhibiteur de PDE4B, un composé de la famille des pyrazolopyridines, parmi lesquels figure notamment l'étazolate.

Les composés de la famille des pyrazolopyridines sont en particulier choisis parmi les composés suivants :
L'étazolate de formule suivante : 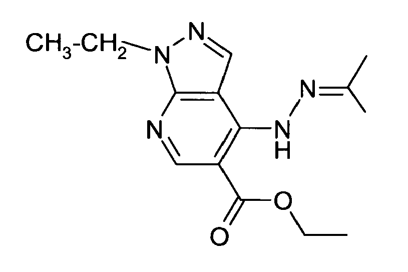
Ester éthylique de l'acide 4-butylamino-1-ethyl-6-methyl-1*H*-pyrazolo[3,4-*b*]pyridine-5-carboxylique (tracazolate),
Ester éthylique de l'acide 4-butylamino-1-ethyl-1*H*-pyrazolo[3,4-b]pyridine-5-carboxylique
1-(4-amino-pyrazolo[3,4-*b*]pyridin-1-yl)-β-*D*-1-deoxy-ribofuranose
Ester éthylique de l'acide 1-ethyl-4-(N'-isopropylidene-hydrazino)-1H-pyrazolo[3,4-b]pyridine-5-carboxylique (SQ 20009),
4-amino-6-methyl-1-n-pentyl-1H-pyrazolo[3,4-b]pyridine
Ester éthylique de l'acide 4-Amino-1-ethyl-6-methyl-1H-pyrazolo[3,4-b]pyridine-5-carboxylique (desbutyl tracacolate),
4-amino-1-pentyl-1H-pyrazolo[3,4-b]pyridine-5-carboxamide,
Ester éthylique de l'acide 1-ethyl-6-methyl-4-methylamino-1*H*-pyrazolo[3,4-b]pyridine-5-carboxylique,
Ester éthylique de l'acide 4-amino-6-methyl-1-propyl-1*H*-pyrazolo[3,4*-b*]pyridine-5-carboxylique,
Ester éthylique de l'acide 1-ethyl-4-ethylamino-6-methyl-1*H*-pyrazolo[3,4-b]pyridine-5-carboxylique,
Ester éthylique de l'acide 4-amino-1-butyl-6-methyl-1*H*-pyrazolo[3,4*-b*]pyridine-5-carboxylique,
5-(4-amino-pyrazolo[3,4-*b*]pyridin-1-yl)-2-hydroxymethyl-tetrahydro-furan-3-ol,
ester allylique de l'acide 1-allyl-4-amino-6-methyl-1*H*-pyrazolo[3,4*-b*]pyridine-5-carboxylique,
acide 4-amino-6-methyl-1-pentyl-1*H*-pyrazolo[3,4-*b*]pyridine-5-carboxylique,
ester éthylique de l'acide 4-amino-1-ethyl-3,6-dimethyl-1*H*-pyrazolo[3,4-b]pyridine-5-carboxylique,
ester éthylique de l'acide 4-dimethylamino-1-ethyl-6-methyl-1*H*-pyrazolo[3,4-b]pyridine-5-carboxylique,
ester éthylique de l'acide 1-ethyl-6-methyl-4-propylamino-1*H*-pyrazolo[3,4-b]pyridine-5-carboxylique,
ester éthylique de l'acide 4-amino-1-pentyl-1*H*-pyrazolo[3,4*-b*]pyridine-5-carboxylique,
ester éthylique de l'acide 4-amino-6-methyl-1-pent-4-ynyl-1*H*-pyrazolo[3,4-b]pyridine-5-carboxylique,
4-amino-1-but-3-enyl-1*H*-pyrazolo[3,4*-b*]pyridine-5-allylamide,
4-amino-1-pentyl-1*H*-pyrazolo[3,4*-b*]pyridine-5-isopropylamide,
4-amino-1-pentyl-N-n-propyl-1*H*-pyrazolo-[3,4-b]pyridine-5-carboxamide,
ester allylique de l'acide 4-amino-1-butyl-6-methyl-1*H*-pyrazolo[3,4*-b*]pyridine-5-carboxylique,
ester éthylique de l'acide 4-amino-6-methyl-1-pent-3-ynyl-1*H*-pyrazolo[3,4-b]pyridine-5-carboxylique,
4-amino-1-pentyl-1*H*-pyrazolo[3,4-b]pyridine-5-prop-2-ynylamide
ester allylique de l'acide 4-amino-1-(3-methyl-butyl)-1*H*-pyrazolo[3,4*-b*]pyridine-5-carboxylique,
4-amino-1-pentyl-1H-pyrazolo<3,4-b>pyridine-5-N-(2-propenyl)carboxamide,
ester allylique de l'acide 4-amino-1-pentyl-1*H*-pyrazolo[3,4*-b*]pyridine-5-carboxylique,
4-amino-1-pentyl-1*H*-pyrazolo[3,4*-b*]pyridine-5-butylamide,
ester allylique de l'acide 4-amino-1-but-3-ynyl-6-methyl-1*H*-pyrazolo[3,4-b]pyridine-5-carboxylique,
ester allylique de l'acide 4-amino-1-but-3-enyl-6-methyl-1*H*-pyrazolo[3,4-b]pyridine-5-carboxylique,
4-amino-6-methyl-1-pentyl-1*H*-pyrazolo[3,4*-b*]pyridine-5-allylamide,
ester allylique de l'acide 4-amino-6-methyl-1-pentyl-1*H*-pyrazolo[3,4-b]pyridine-5-carboxylique,
ester allylique de l'acide 4-amino-6-methyl-1-(3-methyl-butyl)-1*H*-pyrazolo[3,4-*b*]pyridine-5-carboxylique,
ester isobutylique de l'acide 4-amino-6-methyl-1-pentyl-1*H*-pyrazolo[3,4-b]pyridine-5-carboxylique,
4-amino-6-methyl-1-pentyl-1*H*-pyrazolo[3,4*-b*]pyrid ine-5-butylam ide,
ester allylique de l'acide 4-amino-6-methyl-1-(3-methyl-but-2-enyl)-1*H-*pyrazolo[3,4*-b*]pyridine-5-carboxylique,
4-amino-1-pentyl-1*H*-pyrazolo[3,4*-b*]pyridine-5-cyclopropylamide,
ethyl 4-amino-1-pentyl-1H-pyrazolo[3,4-b]pyridine-5-hydroxamate,
ester prop-2-ynylique de l'acide 4-amino-6-methyl-1-pentyl-1*H*-pyrazolo[3,4-b]pyridine-5-carboxylique,
ester allylique de l'acide 4-amino-6-methyl-1-pent-4-ynyl-1*H*-pyrazolo[3,4-b]pyridine-5-carboxylique,
ester allylique de l'acide 4-amino-6-methyl-1-pent-4-enyl-1*H*-pyrazolo[3,4-b]pyridine-5-carboxylique,
4-amino-1-pent-3-ynyl-1*H*-pyrazolo[3,4-*b*]pyridine-5-propylamide,
4-amino-1-pentyl-1*H*-pyrazolo[3,4*-b*]pyridine-5-cyclopropylmethyl-amide,
ester 2-méthyl-allylique de l'acide 4-amino-6-methyl-1-pentyl-1*H*-pyrazolo[3,4-*b*]pyridine-5-carboxylique,
4-Amino-1-pent-3-ynyl-1H-pyrazolo[3,4-b]pyridine-5-allylamide (ICI 190,622),
4-amino-1-pent-4-ynyl-N-2-propenyl-1H-pyrazolo[3,4-*b*]pyridine-5-carboxamide,
4-amino-1-pent-3-ynyl-1*H*-pyrazolo[3,4-ib]pyridine-5-prop-2-ynylamide,
4-amino-1-pentyl-1*H*-pyrazolo[3,4*-b*]pyridine-5-but-2-ynylamide,
ester allylique de l'acide 4-amino-6-methyl-1-pent-3-ynyl-1*H*-pyrazolo[3,4-*b*]pyridine-5-carboxylique,
ester allylique de l'acide 4-amino-1-(2-cyclopropyl-ethyl)-6-methyl-1*H-*pyrazolo[3,4-*b*]pyridine-5-carboxylique,
ester allylique de l'acide 4-amino-1-hex-5-ynyl-6-methyl-1*H*-pyrazolo[3,4-*b*]pyridine-5-carboxylique,
4-amino-1-pent-3-ynyl-1*H*-pyrazolo[3,4-*b*]pyridine-5-cyclopropylmethyl-amide,
ester but-3-énylique de l'acide 4-amino-6-methyl-1-pentyl-1*H*-pyrazolo[3,4-b]pyridine-5-carboxylique,
ester cyclopropylmethylique de l'acide 4-amino-6-methyl-1-pentyl-1H-pyrazolo[3,4*-b*]pyridine-5-carboxylique,
4-butylamino-1-pentyl-1*H*-pyrazolo[3,4-*b*]pyridine-5-allylamide,
ester 2-cyclopropyl-ethylique de l'acide 4-amino-6-methyl-1-pentyl-1*H-*pyrazolo[3,4*-b*]pyridine-5-carboxylique,
ester cyclopropylmethylique de l'acide 4-amino-6-methyl-1-pent-3-ynyl-1*H-*pyrazolo[3,4*-b*]pyridine-5-carboxylique,
ester cyclopropylmethylique de l'acide 4-amino-6-methyl-1-pent-4-ynyl-1*H-*pyrazolo[3,4-*b*]pyridine-5-carboxylique,
ester éthylique de l'acide 4-amino-1-benzyl-6-methyl-1*H*-pyrazolo[3,4*-b*]pyridine-5-carboxylique,
4-amino-1-pentyl-1*H*-pyrazolo[3,4*-b*]pyridine-5-benzylamide,
4-amino-1-pentyl-1*H*-pyrazolo[3,4*-b*]pyrid ine-5-phenylam ide,
ester benzylique de l'acide 4-amino-6-methyl-1-pentyl-1*H*-pyrazolo[3,4-b]pyridine-5-carboxylique,
4-Azido-1-β-D-ribofuranosylpyrazolo[3,4-b]pyridine.
1-pent-3-ynyl-N-2-propenyl-4-propionamido-1H-pyrazolo[3,4-b]pyridine-5-carboxamide,
2-(4-amino-pyrazolo[3,4*-b*]pyridin-1-yl)-5-hydroxymethyl-tetrahydro-furan-3,4-diol,
2-(6-methyl-1*H*-pyrazolo[3,4*-b*]pyridin-4-ylamino)-ethanol,
3-(6-methyl-1*H*-pyrazolo[3,4-*b*]pyridin-4-ylamino)-propan-1-ol,
ester propylique de l'acide 3-(6-methyl-1*H*-pyrazolo[3,4*-b*]pyridin-4-ylamino)-acétique,
ester éthylique de l'acide 2-(6-methyl-1*H*-pyrazolo[3,4*-b*]pyridin-4-ylamino)-propionique,
ester éthylique de l'acide 2-(6-methyl-1*H*-pyrazolo[3,4*-b*]pyridin-4-ylamino)-pentanoique,
ester éthylique de l'acide 2-(6-methyl-1*H*-pyrazolo[3,4*-b*]pyridin-4-ylamino)-benzoique,
ester propylique de l'acide 3-(6-methyl-1*H-*pyrazolo[3,4-*b*]pyridin-4-ylamino)-pentanoïque,
*N*-benzylidene-*N*'-(3-methyl-1-phenyl-1*H*-pyrazolo[3,4*-b*]pyridin-4-yl)-hydrazine,
*N*-furan-2-ylmethylene-*N*'-(3-methyl-1-phenyl-1*H*-pyrazolo[3,4*-b*]pyridin-4-yl)-hydrazine,
*N-*(4-fluoro-benzylidene)-*N'*-(3-methyl-1-phenyl-1*H*-pyrazolo[3,4*-b*]pyridin-4-yl)-hydrazine,
*N-*(3-furan-2-yl-allylidene)-*N'*-(3-methyl-1-phenyl-1*H*-pyrazolo[3,4*-b*]pyridin-4-yl)-hydrazine,
*N-*(4-methoxy-benzylidene)-*N'*-(3-methyl-1-phenyl-1*H*-pyrazolo[3,4*-b*]pyridin-4-yl)-hydrazine,
4-[(3-methyl-1-phenyl-1*H*-pyrazolo[3,4-*b*]pyridin-4-yl)-hydrazonomethyl]-benzonitrile,
*N*-benzo[1,3]dioxol-5-ylmethylene-*N'*-(3-methyl-1-phenyl-1*H*-pyrazolo[3,4-*b*]pyridin-4-yl)-hydrazine,
*N*-(3-methyl-1-phenyl-1*H*-pyrazolo[3,4-*b*]pyridin-4-yl)-*N*'-(4-nitro-benzylidene)-hydrazine,
*N*-(3-methyl-1-phenyl-1*H*-pyrazolo[3,4*-b*]pyridin-4-yl)-*N*'-(2-nitro-benzylidene)-hydrazine,
*N-*(3-methyl-1-phenyl-1*H*-pyrazolo[3,4*-b*]pyridin-4-yl)-*N*'-(4-trifluoromethyl-benzyl idene)-hydrazine,
*N-*(3-methyl-1-phenyl-1*H*-pyrazolo[3,4*-b*]pyridin-4-yl)-*N*'-(5-nitro-furan-2-ylmethylene)-hydrazine,
*N-*(3-methyl-1-phenyl-1*H*-pyrazolo[3,4*-b*]pyridin-4-yl)-*N'*-(2-trifluoromethyl-benzyl idene)-hydrazine,
*N-*(3-methyl-1-phenyl-1*H*-pyrazolo[3,4*-b*]pyridin-4-yl)-N'-(6-nitro-benzo[1,3]dioxol-5-ylmethylene)-hydrazine,
Acide 4-(3-chloro-4-methoxy-benzylamino)-1-ethyl-1*H*-pyrazolo[3,4*-b*]pyridine-5-carboxylique,
4-(3-chloro-4-methoxy-benzylamino)-1-ethyl-1*H*-pyrazolo[3,4*-b*]pyridine-5-(pyridin-4-ylmethyl)-amide,
4-(3-chloro-4-methoxy-benzylamino)-1-ethyl-1*H*-pyrazolo[3,4*-b*]pyridine-5-(tetrahydro-furan-2-ylmethyl)-amide,
4-(3-chloro-4-methoxy-benzylamino)-1-ethyl-1*H*-pyrazolo[3,4-*b*]pyridine-5-(5-hydroxy-pentyl)-amide,
4-(3-chloro-4-methoxy-benzylamino)-1-ethyl-1*H*-pyrazolo[3,4*-b*]pyridine-5-[3-(2-oxo-pyrrolidin-1-yl)-propyl]-amide,
ester éthylique de l'acide 4-*tert*-butylamino-1-(2-chloro-2-phenyl-ethyl)-1*H-*pyrazolo[3,4*-b*]pyridine-5-carboxylique,
ester éthylique de l'acide 1-(2-chloro-2-phenyl-ethyl)-4-cyclopropylamino-1*H-*pyrazolo[3,4*-b*]pyridine-5-carboxylique,
ester éthylique de l'acide 1-(2-chloro-2-phenyl-ethyl)-4-propylamino-1*H-*pyrazolo[3,4*-b*]pyridine-5-carboxylique,
ester éthylique de l'acide 1-(2-chloro-2-phenyl-ethyl)-4-phenylamino-1*H-*pyrazolo[3,4*-b*]pyridine-5-carboxylique,
ester éthylique de l'acide 4-butylamino-1-(2-chloro-2-phenyl-ethyl)-1*H-*pyrazolo[3,4*-b*]pyridine-5-carboxylique,
ester éthylique de l'acide 1-(2-chloro-2-phenyl-ethyl)-4-(2-ethoxy-ethylamino)-1*H*-pyrazolo[3,4*-b*]pyridine-5-carboxylique,
ester éthylique de l'acide 4-benzylamino-1-(2-chloro-2-phenyl-ethyl)-1*H-*pyrazolo[3,4*-b*]pyridine-5-carboxylique,
ester éthylique de l'acide 1-(2-chloro-2-phenyl-ethyl)-4-phenethylamino-1*H-*pyrazolo[3,4*-b*]pyridine-5-carboxylique,

La présente invention propose donc, pour la première fois, la PDE4B comme cible thérapeutique pour le traitement des événements moléculaires associés à l'excitotoxicité. Selon des modes de mise en oeuvre particuliers, l'invention peut être utilisée pour inhiber ou réduire l'excitotoxicité neuronale en phase précoce des maladies neurodégénératives. Elle est applicable donc au traitement de l'ALS.

D'autres objets de l'invention résident dans :
. l'utilisation des composés indiqués ci-dessus, en particulier de l'étazolate, pour le traitement de l'ALS, notamment pour réduire l'excitotoxicité neuronale en phase précoce de l'ALS, ou
. l'utilisation des composés indiqués ci-dessus, en particulier de l'étazolate, pour la préparation d'une composition destinée à inhiber l'activité de la PDE4B chez les patients atteints d'ALS.

L'invention concerne également des méthodes de traitement de l'ALS comprenant l'administration d'un composé inhibant sélectivement l'expression ou l'activité de la PDE4B de séquence SEQ ID n° 2 ou 4. De préférence, les méthodes de l'invention sont utilisées pour le traitement en phase précoce de l'ALS.

L'administration peut être réalisée par toute méthode connue de l'homme du métier, de préférence par voie orale ou par injection, typiquement par voie intra-péritonéale, intra-cérébrale, intra-veineuse, intra-artérielle ou intra-musculaire. L'administration par voie orale est préférée. Les doses administrées peuvent être adaptées par l'homme de l'art. Typiquement, de 0,01 mg à 100 mg / kg environ sont injectés, pour des composés inhibiteurs de nature chimique. Pour des composés nucléiques, les doses peuvent varier par exemple entre 0,01 mg et 100 mg par dose. Il est entendu que des injections répétées peuvent être réalisées, éventuellement en combinaison avec d'autres agents actifs ou tout véhicule acceptable sur le plan pharmaceutique (ex., tampons, solutions saline, isotonique, en présence d'agents stabilisants, etc.).

L'invention est utilisable chez les mammifères, notamment chez l'être humain. Les résultats présentés dans les exemples illustrent l'efficacité d'inhibiteurs de PDE4B pour améliorer la viabilité de neurones placés en conditions d'excitotoxicité.

### Méthodes de sélection et outils

L'invention divulgue aussi des méthodes de sélection, identification ou caractérisation de composés actifs sur les pathologies associées à l'excitotoxicité, ou au stress neuronal, comprenant la mise en contact de composés tests avec une cellule exprimant la PDE4B (notamment un variant dépourvu de domaine 3' non-codant), et la mise en évidence des composés inhibant l'expression ou l'activité de cette protéine.

Les méthodes peuvent être mises en oeuvre avec différentes populations cellulaires, telles que des cellules primaires ou des lignées de cellules d'origine mammifère (humaine, murine, etc.). On utilise avantageusement des cellules qui n'expriment pas naturellement la PDE4B, transfectées avec un acide nucléique codant le variant souhaité. De cette manière, la sélectivité de la méthode est augmentée. On peut également utiliser des cellules eucaryotes inférieures (levure, etc.) ou des cellules procaryotes.

Les méthodes de screening peuvent également être réalisées en système acellulaire, par mesure de la capacité de composés tests à lier la PDE4B ou un variant ou fragment de celle-ci.

Un autre objet de l'invention concerne tout acide nucléique codant un polypeptide tel que défini ci-dessus, les vecteurs le contenant, cellules recombinantes, et utilisations. Les vecteurs peuvent être des plasmides, phages, cosmides, virus, chromosomes artificiels, etc. Des vecteurs préférés sont par exemple des vecteurs plasmidiques, comme ceux dérivés de plasmides commerciaux (pUC, pcDNA, pBR, etc.). De tels vecteurs comportent avantageusement un gène de sélection et/ou une origine de réplication et/ou un promoteur transcriptionnel. D'autres vecteurs particuliers sont par exemples des virus ou des phages, notamment des virus recombinants défectifs pour la réplication, tels que des virus dérivés de rétrovirus, adénovirus, AAV, herpès-virus, baculovirus, etc. Les vecteurs peuvent être utilisés dans tout hôte compétent, comme par exemple des cellules prokaryotes ou eukaryotes. Il peut s'agit de bactéries (par exemple E. coli), levures (par exemple Saccharomyces ou Kluyveromyces), cellules végétales, cellules d'insectes, cellules de mammifères, notamment humaines, etc. Il peut s'agir de lignées, cellules primaires, cultures mixtes, etc.

D'autres aspects et avantages de la présente invention apparaîtront à la lecture des exemples qui suivent, qui doivent être considérés comme illustratifs et non limitatifs.

### LEGENDE DES FIGURES

Figure 1: PCR semi-quantitative de PDE4B à partir d'échantillons de cerveau (1A) et de muscle (1B).
Figure 2: La pentoxifylline protège les neurones primaires de granules cérébelleux de l'excitotoxicité induite par le kainate.
Figure 3: La pentoxifylline protège les neurones primaires de granules cérébelleux de l'excitotoxicité induite par NMDA/serine.
Figure 4: Effet neuroprotecteur de l'étazolate sur la toxicité induite par NMDA/serine sur les cellules granulaires du cervelet.
Figure 5: Effet neuroprotecteur de l'étazolate sur la toxicité induite par le kainate sur les cellules granulaires du cervelet.
Figure 6: Effet neuroprotecteur de la pentoxifylline sur la toxicité induite par NMDA/serine sur les neurones corticaux.
Figure 7: Effet neuroprotecteur de la pentoxifylline sur la toxicité induite par le kainate sur les neurones corticaux.
Figure 8: Effet neuroprotecteur de l'étazolate sur la toxicité induite par NMDA/serine sur les neurones corticaux.
Figure 9: Effet neuroprotecteur de l'étazolate sur la toxicité induite par le kainate sur les neurones corticaux.
Figure 10: Effet neuroprotecteur du 8-bromo-cAMP sur la toxicité induite par NMDA/serine sur les cellules granulaires du cervelet.
Figure 11: Effet neuroprotecteur du 8-bromo-cAMP sur la toxicité induite par le kainate sur les cellules granulaires du cervelet.

### EXEMPLES

### Exemple 1 : Identification de la PDE4 comme cible moléculaire de l'excitotoxicité

L'analyse qualitative différentielle a été effectuée à partir d'ARN poly adénylés (poly A+) extraits d'échantillons de cerveaux d'animaux correspondant aux différents stades, sans isolement préalable des neurones afin de prendre en compte un maximum d'évènements d'épissages alternatifs liés au développement de la pathologie.
Les ARN poly A+ sont préparés selon des techniques connues de l'homme de métier. Il peut s'agir en particulier d'un traitement au moyen d'agents chaotropiques tels que le thiocyanate de guanidium suivi d'une extraction des ARN totaux au moyen de solvants (phénol, chloroforme par exemple). De telles méthodes sont bien connues de l'homme du métier (voir Maniatis et al., Chomczynsli et al., Anal. Biochem. 162 (1987) 156), et peuvent être aisément pratiquées en utilisant des kits disponibles dans le commerce. A partir de ces ARN totaux, les ARN poly A+ sont préparés selon des méthodes classiques connues de l'homme de métier et proposées par des kits commerciaux.
Ces ARN poly A+ servent de matrice à des réactions de transcription inverse à l'aide de reverse transcriptase. Avantageusement sont utilisées des reverse transcriptases dépourvues d'activité RNase H qui permettent d'obtenir des premiers brins d'ADN complémentaire de tailles supérieures à ceux obtenus avec des reverse transcriptases classiques. De telles préparations de reverse transcriptases sans activité RNase H sont disponibles commercialement.
Pour chaque point de la cinétique de développement de la pathologie (30 jours, 60 jours et 90 jours) les ARN poly A+ ainsi que les ADNc simple brins sont préparés à partir des animaux transgéniques (T) et des animaux contrôles syngéniques (C).
Conformément à la technique DATAS, pour chaque point de la cinétique sont réalisées des hybridations d'ARNm (C) avec des ADNc (T) et des hybridations réciproques d'ARNm (T) avec des ADNc (C).
Ces hétéroduplex ARNm/ADNc sont ensuite purifiés selon les protocoles de la technique DATAS.

Les séquences d'ARN non appariées avec un ADN complémentaire sont libérées de ces hétéroduplex sous l'action de la RNase H, cette enzyme dégradant les séquences d'ARN appariées. Ces séquences non appariées représentent les différences qualitatives qui existent entre des ARN par ailleurs homologues entre eux. Ces différences qualitatives peuvent être localisées n'importe où sur la séquence des ARN, aussi bien en 5', 3' ou à l'intérieur de la séquence et notamment dans la séquence codante. Selon leur localisation, ces séquences peuvent être non seulement des modifications d'épissage mais également des conséquences de translocations ou de délétions.
Les séquences d'ARN représentant les différences qualitatives sont ensuite clonées selon les techniques connues de l'homme de métier et notamment celles décrites dans le brevet de la technique DATAS.
Ces séquences sont regroupées au sein de banques de cDNA qui constituent des banques qualitatives différentielles. Une de ces banques contient les exons et les introns spécifiques de la situation saine ; les autres banques contiennent les évènements d'épissage caractéristiques des conditions pathologiques.
L'expression différentielle des clones a été vérifiée par hybridation avec des sondes obtenues par reverse-transcription à partir d'ARN messagers extraits des différentes situations étudiées. Les clones hybridant de façon différentielle ont été retenus pour analyse ultérieure. Les séquences identifiées par DATAS correspondent à des introns et/ou à des exons exprimées de façon différentielle par épissage entre les situations pathologiques et la situation saine. Ces évènements d'épissage peuvent être spécifiques d'une étape donnée du développement de la pathologie ou caractéristiques de l'état sain.

La comparaison de ces séquences avec les banques de données permet de classifier les informations obtenues et de proposer une sélection raisonnée des séquences selon leur intérêt diagnostique ou thérapeutique.

La réalisation de DATAS sur des ARN d'animaux contrôles et transgéniques âgés de 60 jours a permis d'isoler un fragment d'ADNc dérivé de l'ARNm de la phosphodiestérase 4B. Ce fragment correspond à un fragment d'exon spécifiquement présent dans les animaux contrôles et donc spécifiquement délété dans les animaux transgéniques pour SOD1 G93A au stade 60 jours. Ce fragment recouvre les nucléotides 377 à 486 référencés à partir du codon stop de la PDE4B de souris (SEQ ID NO :1). Cette séquence comprend 2912 bases, le fragment délété correspondant aux bases 2760 à 2869. Cette région est non codante et est exprimée différentiellement entre les animaux contrôles et les animaux transgéniques, du fait de l'utilisation alternative d'un exon 3' non codant ou du fait de l'utilisation de deux sites de polyadénylation alternatifs.

### Exemple 2 : Expériences de RT-PCR : Confirmation de l'expression différentielle :

L'expression différentielle de la PDE4B dans une situation de stress neuronal, par rapport à une situation de référence, a été vérifiée par des expériences de RT PCR présentées sur la figure 1.
Ces expériences ont été réalisées selon des techniques bien connues de l'homme de métier et ont permis de suivre les expressions de deux régions distinctes de l'ARNm de la PDE4B. Une de ces régions recouvre le codon d'initiation de cet ARNm (PDE4B 5'), l'autre recouvre en partie le fragment identifié selon la technique DATAS (PDE4B DATAS). Les localisations des amorces de PCR utilisées sont indiquées sur la figure 1.
L'ARN PO correspond à un ARN ribosomal utilisé comme contrôle interne destiné à vérifier que la même quantité d'ARN est utilisée pour chaque point expérimental. Les analyses ont été réalisées à partir d'ARN extraits d'animaux contrôles (C) et transgéniques (T) âgés de 30, 60 et 90 jours, c'est à dire avant l'apparition des symptômes pathologiques.
Les ARN totaux du cerveau des souris contrôle ou SOD1 G93A âgées 30, 60 et 90 jours sont transcrits en ADNc utilisant le protocole standard de Superscript^{™} (Invitrogen). Pour les PCR semi-quantitatives les produits de la réaction de reverse transcription sont dilués 10 fois. Les amorces spécifiques du fragment DATAS correspondent pour le sens aux nucléotides 2526-2545 (5' GCC AGG CCG TGA AGC AAA TA 3' ; SEQ ID NO : 5), et pour l'anti-sens aux 2790-2807 (5' TCA AAG ACG CGA AAA CAT 3'; SEQ ID NO : 6) et pour le fragment plus en 3 prime les amorces correspondent pour le sens aux nucléotides 145-165 (5' CCG CGT CAG TGC CTT TGC TAT 3'; SEQ ID NO : 7), et pour l'anti-sens aux 426-404 (5' CGC TGT CGG ATG CTT TTA TTC AC 3'; SEQ ID NO : 8). Comme gène de référence le gène P0 est utilisé et amplifié par les amorces, sens : 5' TCG CTT TCT GGA GGG TGT C 3' (SEQ ID NO : 9) et anti-sens : CCG CAG GGG CAG CAG TGG 3' (SEQ ID NO :10).
L'amplification est effectuée par 30 cycles de PCR suivants :
- 30 secondes à 94°C
- une minute à 57°C
- 30 secondes à 72°C, suivi par un cycle de 2 minutes à 72°C
Les différents produits de PCR sont mis sur un gel d'agarose de 1.5 %. L'expérience est répétée trois fois avec deux réactions de reverse transcription différentes.

La figure 1 présente les résultats obtenus à partir d'ARN extraits des cerveaux ou des muscles des animaux.
Alors que la même quantité d'ADNc est amplifiée à partir de l'ARN de PO dans tous les échantillons, de variations sont observées pour l'ARNm de la PDE4B : les variations les plus significatives sont détectées chez les animaux âgés de 90 jours : alors qu'une augmentation du niveau d'expression du fragment PDE4 5' est observée dans le cerveau des animaux transgéniques, une très forte diminution de l'expression de PDE4B (DATAS) est observée dans le cerveau des animaux transgéniques.
Ce résultat établit une corrélation entre la diminution de l'expression d'un fragment 3' non codant de l'ARNm de la PDE4B et l'augmentation de l'expression de la partie 5' codante de ce même messager. Ce résultat est tout à fait compatible avec la présence de séquences de déstabilisation des ARNm dans la séquence identifiée par DATAS et démontre la corrélation entre l'expression de PDE4B et le phénomène d'excitotoxicité.

### Exemple 3 : Inhibition de l'excitotoxicité par des inhibiteurs de PDE4

Pour cet exemple, des neurones granulaires du cervelet de rat ainsi que des neurones corticaux ont été mis en culture selon les techniques connues de l'homme de métier.

### Culture primaire des cellules granulaires de cervelet :

Les rats Wistar âgés de sept jours sont décapités et leurs cervelets sont disséqués. Après avoir enlevé les méninges, le tissu est coupé en petits morceaux et trypsinisé pendant 15 minutes à 37°C. Les cellules sont dissociées par trituration et mises en cultures à une densité 300.000 cellules par cm² dans du milieu basal Eagle supplémenté avec 10% du sérum de veau foetal et 2 mM glutamine. Le lendemain 10 µM ARA-C, un anti-mitotique, est ajouté pour empêcher la prolifération des cellules gliales. Les cellules sont traitées le jour 9 de cultures avec les phosphodiesterase inhibiteurs, pentoxifylline et étazolate, trois heures avant l'addition des toxiques, 50 µM kainate ou 100 µM N-methyl-D-aspartate en présence de 10 µM D-sérine. Le 8-bromo-cAMP est ajouté juste avant les toxiques. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### Cultures primaires des cellules corticales :

Des embryons de rat Wistar, âgés de 16 jours, sont prélevés et les cortex sont disséqués. Après la trypsination à 37°C pendant 25 minutes, les cellules sont dissociées par trituration. Les cellules sont ensemencées dans du milieu essentiel minimum, supplémenté avec 10% de sérum de cheval et 10% de sérum de veau foetal et 2 mM glutamine, à une densité de 300.000 cellules par cm². Après 4 jours en culture la moitié du milieu est changée avec du milieu essentiel minimum supplémenté avec 5% de sérum de cheval et 2 mM glutamine. Le même jour, 10 µM de 5-fluoro-2-deoxyuridine, un anti-mitotique, est ajouté. Après sept et onze jours de culture, la moitié du milieu est changée par du milieu conditionné. Le milieu conditionné est composé de MEM contenant 5 % de sérum de cheval et 2 mM glutamine ; ce milieu est passé sur un tapis d'astrocytes corticales pendant une nuit avant son utilisation. A jour 14, les cellules sont traitées avec les phosphodiesterase inhibiteurs, pentoxifylline et étazolate, une heure avant l'addition des toxiques, 50 µM kainate ou 20 µM N-methyl-D-aspartate en présence de 10 µM D-sérine. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### MTT:

La toxicité est mesurée en utilisant le test MTT. Après l'incubation avec les composés, du MTT est ajouté à une concentration finale de 0.5 mg/ml par puits. Les plaques sont ensuite incubées pendant 30 minutes à 37 °C dans le noir. Le milieu est aspiré et les cristaux sont resuspendus dans 500 µl de DMSO (dimethylsulfoxyde). L'absorbance à 550 nm est lue et le pourcentage de viabilité est calculé.

### Résultats :

Les résultats obtenus sont présentés sur les figures 2-10. Ces résultats illustrent l'effet protecteur des composés de l'invention sur la survie neuronale. Lors du co-traitement des neurones par un inhibiteur de PDE4, un effet protecteur dose-dépendent est observé dans les deux modes d'induction de l'excitotoxicité (NMDA/Serine et kainate). Un tel effet protecteur est observé à l'aide de la pentoxifylline et de l'étazolate.

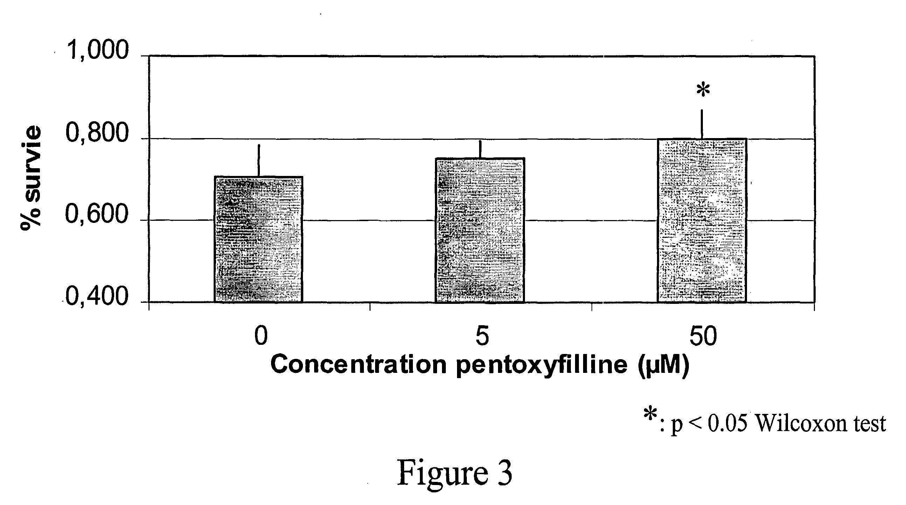

Les figures 2 et 3 présentent des résultats obtenus à l'aide de la pentoxifylline sur les cellules granulaires du cervelet. Les résultats présentés montrent que la pentoxifylline permet d'atteindre sur ces cellules un effet protecteur de 43% dans le cas du traitement NMDA/serine, et de 33% dans le cas de la toxicité induite par le kainate.

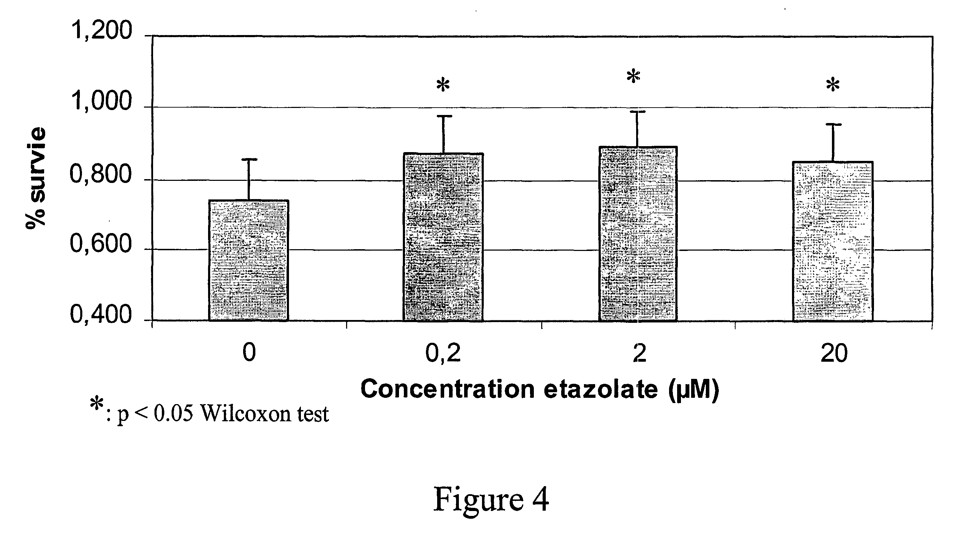

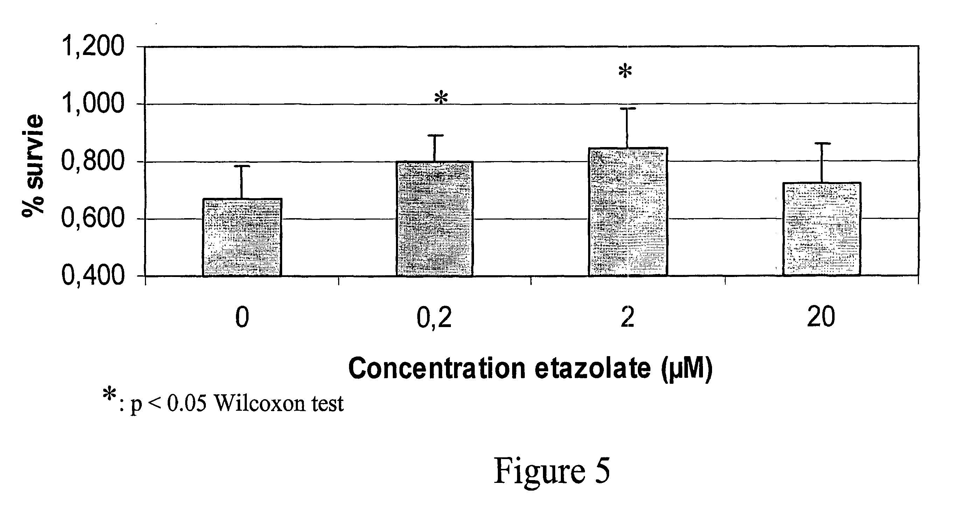

Les figures 4 et 5 présentent des résultats obtenus à l'aide de l'étazolate sur les cellules granulaires du cervelet. Les résultats présentés montrent que l'étazolate permet d'atteindre sur ces cellules un effet protecteur de 60% dans le cas du traitement NMDA/Ser, et de 57% dans le cas de la toxicité induite par le kainate.

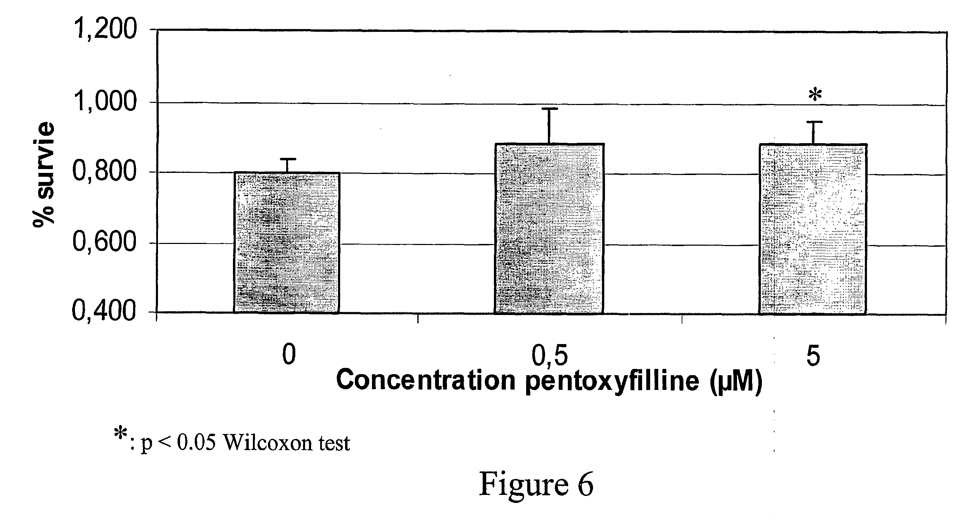

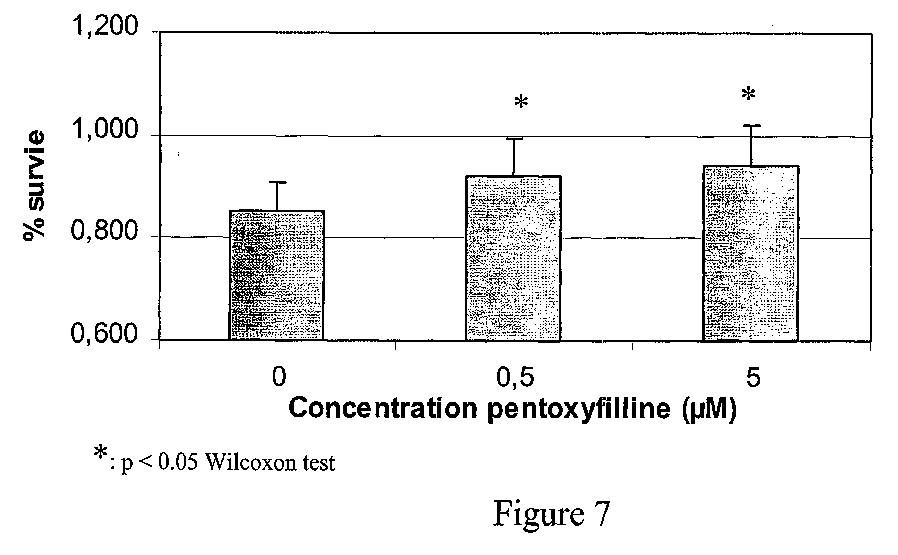

Les figures 6 et 7 présentent des résultats obtenus à l'aide de la pentoxifylline sur les neurones corticaux. Les résultats présentés montrent que la pentoxifylline permet d'atteindre sur ces cellules un effet protecteur de 50% dans le cas du traitement NMDA/Ser, et de 66% dans le cas de la toxicité induite par le kainate.

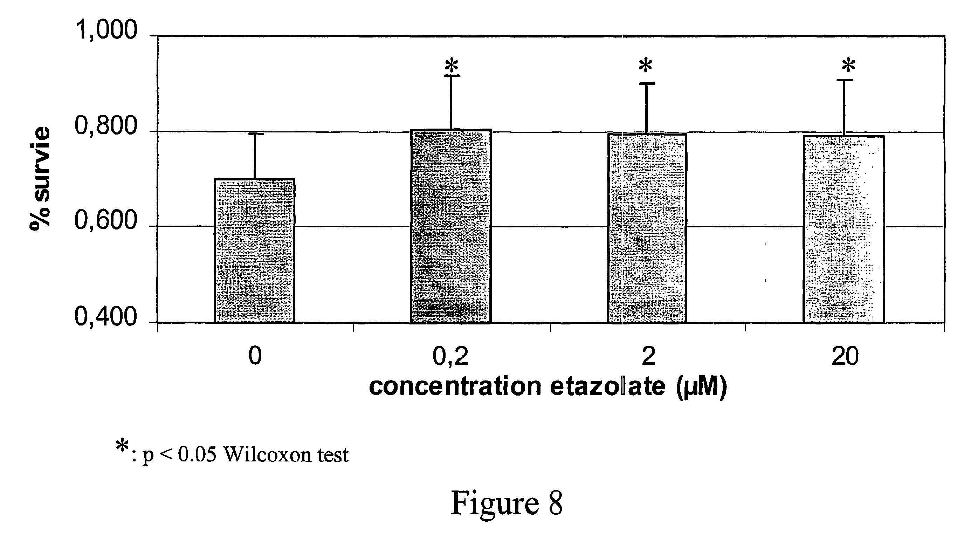

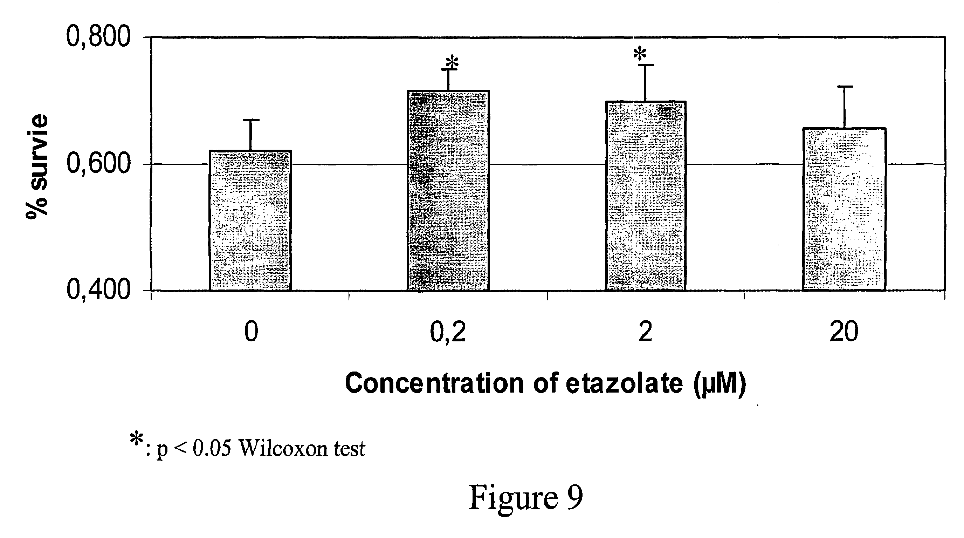

Les figures 8 et 9 présentent des résultats obtenus à l'aide de l'étazolate sur les neurones corticaux. Les résultats présentés montrent que l'étazolate permet d'atteindre sur ces cellules un effet protecteur de 33% dans le cas du traitement NMDA/Ser, et de 25% dans le cas de la toxicité induite par le kainate.

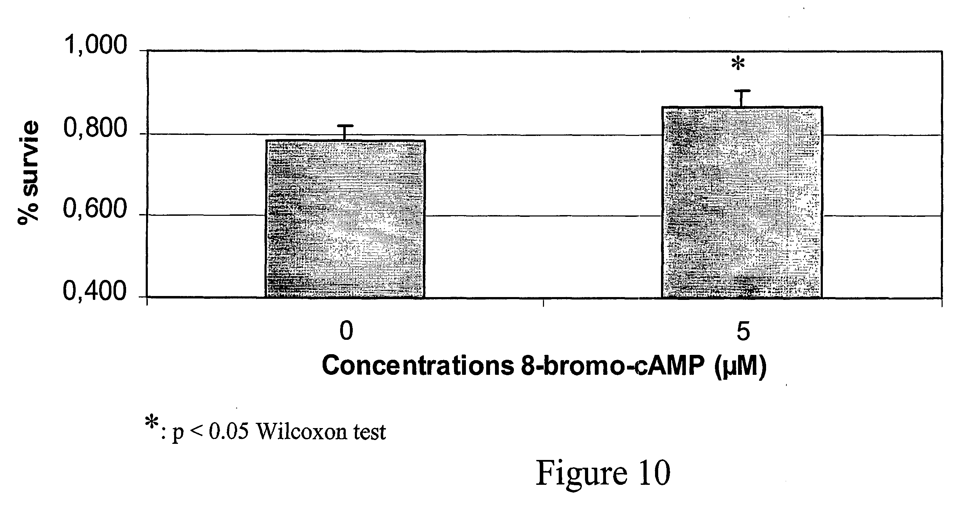

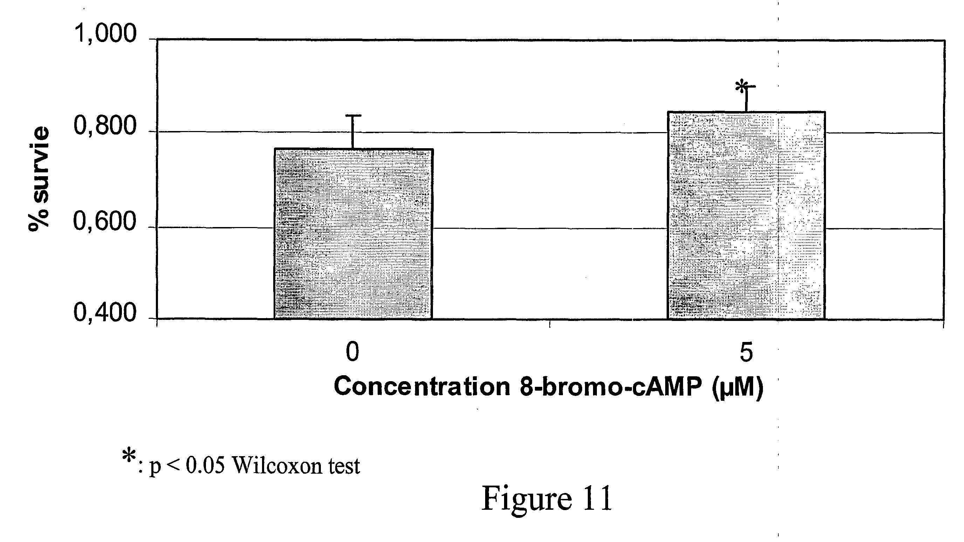

La pertinence de ces protections est attestée par les % de protection obtenus par des concentrations croissantes d'AMPc, substrat des PDE, donnés à titre d'exemple pour les cellules granulaires du cervelet sur les figures 10 et 11. Ces % sont de 40% pour le traitement NMDA/serine et de 40% pour le traitement kainate.

La présente invention documente donc non seulement l'implication de la PDE4B dans les mécanismes d'excitotoxicité, notamment dans un modèle d'ALS, mais également la capacité d'inhibiteurs de PDE4 à préserver la viabilité neuronale lors de stress liés à l'excitotoxicité.

### Exemple 4 : Utilisation clinique chez l'homme

Cet exemple décrit les conditions d'une utilisation en clinique humaine d'un inhibiteur de PDE4, dans le traitement de l'ALS. Cet exemple illustre le potentiel thérapeutique de l'invention et ses conditions d'application à l'être humain.

Dans cet essai clinique, le traitement est basé sur une combinaison de pentoxifylline et de riluzole. La dose de pentoxifylline utilisée est de 400 mg par administration, sous forme de trois prises quotidiennes, soit une dose quotidienne totale de 1200 mg. La pentoxifylline est utilisée sous forme de comprimés. L'essai est multi-centrique et réalisé en double-aveugle contre placebo sur 400 patients. Les patients inclus dans l'essai sont des hommes ou des femmes âgés de 18 à 80 ans, atteints d'ALS sporadique ou familiale, et sous traitement par le riluzole (50 mg b.i.d.) depuis 3 mois au moins. Le traitement à la pentoxifylline est prévu sur une durée de 18 mois.

L'efficacité est mesurée principalement par le taux de survie des patients, la qualité de vie, des tests musculaires.

D'autres aspects et applications de l'invention résident dans :
- l'utilisation de tout ou partie d'une séquence dérivée de l'ARN messager de la PDE4B à des fins de diagnostic ou de dépistage ou de caractérisation de pathologies neurodégénératives ayant une composante ou un stade lié au phénomène d'excitotoxicité, telles que la maladie d'Alzheimer, la maladie de Parkinson, la sclérose en plaques, la chorée de Huntington, l'ALS ou l'ischémie cérébrale,
- l'utilisation de tout fragment d'acide nucléique y compris des ARN anti-sens dans le but d'inhiber l'expression de la PDE4B e chez les patients atteints de telles pathologies,
- l'utilisation de tout composé chimique, notamment de la pentoxifylline, de l'étazolate, ou de toute composition pharmaceutique les contenant, dans le but d'inhiber l'activité de la PDE4B chez les patients atteints de telles pathologies,
- l'utilisation de tout ou partie d'une séquence dérivée de l'ARN messager de la PDE4B à des fins de caractérisation du tissu et de la situation ischémique.

## Revendications

1. Utilisation d'au moins un composé inhibiteur de PDE4B appartenant à la famille des pyrazolopyridines, pour la préparation d'une composition pharmaceutique destinée au traitement de l'ALS.

2. Utilisation selon la revendication 1, **caractérisée en ce que** le composé est l'étazolate.

3. Utilisation selon l'une des revendications 1 ou 2, pour inhiber ou réduire l'excitotoxicité neuronale en phase précoce de l'ALS.

4. Utilisation selon l'une des revendications 1 à 3, pour améliorer la survie neuronale chez les patients atteints d'ALS.

5. Utilisation selon l'une des revendications 1 à 4, pour réduire l'excitotoxicité neuronale en phase précoce de l'ALS.

6. Utilisation de l'étazolate pour la préparation d'une composition destinée à augmenter la survie neuronale chez les patients atteints d'ALS.

## Claims

1. A use of at least one PDE4B inhibiting compound of the pyrazolopyridine family for the preparation of a pharmaceutical composition for the treatment of ALS.

2. The use according to claim 1, **characterized in that** said compound is etazolate.

3. The use according to claim 1 or 2, for the inhibition or reduction of neuronal excitotoxicity in the early stage of ALS.

4. The use according to anyone of claims 1 to 3, for increasing neuron survival in patients with ALS.

5. The use according to anyone of claims 1 to 4, for the reduction of neuronal excitotoxicity in the early stage of ALS.

6. A use of etazolate for the preparation of a composition for increasing neuron survival in patients with ALS.

## Patentansprüche

1. Verwendung von wenigstens einer PDE4B hemmenden Verbindung, die zur Familie der Pyrazolopyridine gehört, für die Zubereitung einer pharmazeutischen Zusammensetzung für die Behandlung der ALS.

2. Verwendung gemäß Anspruch 1, **dadurch gekennzeichnet, dass** die Verbindung Etazolat ist.

3. Verwendung gemäß einem der Ansprüche 1 oder 2 zur Hemmung oder Verminderung der neuronalen Exzitotoxizität in einer frühen Phase der ALS.

4. Verwendung gemäß einem der Ansprüche 1 bis 3 zur Verbesserung des neuronalen Überlebens bei Patienten, die an ALS erkrankt sind.

5. Verwendung gemäß einem der Ansprüche 1 bis 4 zur Verminderung der neuronalen Exzitotoxizität in einer frühen Phase der ALS.

6. Verwendung von Etazolat für die Zubereitung einer Zusammensetzung für eine Steigerung des neuronalen Überlebens bei Patienten, die an ALS erkrankt sind.


### LISTE DE SEQUENCES

<110> Exonhit Therapeutics
<120> Nouvelle cible moleculaire de la Neurotoxicité
<130> B0100WO
<140>
   <141>
<160> 10
<170> PatentIn Ver. 2.1
<210> 1
   <211> 2912
   <212> ADN
   <213> souris
<220>
   <221> CDS
   <222> (218)..(2383)
<400> 1
   aaaggcagcc tgataaagct ccttgtgaca ggctgtcttg ccagtctccc agtatgctcc 60
   tcttgctctg aagtgctcca ggattgaaac cacagcttcc caaattagcc tgggaagagt 120
   gtgcggaccc agcagccttt taacccgcgt cagtgccttt gctatgttca agactgctgt 180
   tttggatggt gaatgctagc tagcactcca tcgagac atg aca gca aaa aat tct  235
                                           Met Thr Ala Lys Asn Ser
                                           1               5
   cca aaa gaa ttt act gct tcg gaa tct gag gtt tgc ata aag act ttc  283
   Pro Lys Glu Phe Thr Ala Ser Glu Ser Glu Val Cys Ile Lys Thr Phe
               10                  15                  20
   aag gag cag atg cgc ttg gaa ctt gag ctt cca aag cta cca gga aac  331
   Lys Glu Gln Met Arg Leu Glu Leu Glu Leu Pro Lys Leu Pro Gly Asn
           25                  30                  35
   aga cct aca tct ccc aaa att tct cca cgc agt tca cca agg aat tca  379
   Arg Pro Thr Ser Pro Lys Ile Ser Pro Arg Ser Ser Pro Arg Asn Ser
       40                  45                  50
   cca tgc ttt ttc aga aag ttg ctg gtg aat aaa agc atc cga cag cgg  427
   Pro Cys Phe Phe Arg Lys Leu Leu Val Asn Lys Ser Ile Arg Gln Arg
    55                  60                  65                  70
   cgt cgc ttc acg gtg gct cat aca tgc ttt gat gtg gaa aat ggc cct    475
   Arg Arg Phe Thr Val Ala His Thr Cys Phe Asp Val Glu Asn Gly Pro
                   75                  80                  85
   tct cca ggt cgg agc cca ctg gac cct caa gcc ggc tct tcg tcg gga    523
   Ser Pro Gly Arg Ser Pro Leu Asp Pro Gln Ala Gly Ser Ser Ser Gly
               90                  95                  100
   ctg gta ctt cat gcc gcc ttt cct ggg cac agc cag cgc agg gag tcg    571
   Leu Val Leu His Ala Ala Phe Pro Gly His Ser Gln Arg Arg Glu Ser
           105                 110                 115
   ttc ctc tac gat ctt gac agc gac tat gac ttg tca cca aaa gcg atg    619
   Phe Leu Tyr Asp Leu Asp Ser Asp Tyr Asp Leu Ser Pro Lys Ala Met
       120                 125                 130
   tcc agg aac tca tca ctt ccc agt gag caa cac ggc gat gac ctg att    667
   Ser Arg Asn Ser Ser Leu Pro Ser Glu Gln His Gly Asp Asp Leu Ile
   135                 140                 145                 150
   gtc act cct ttt gcc cag gtt ctt gcc agc ttg cga agt gta aga aac    715
   Val Thr Pro Phe Ala Gln Val Leu Ala Ser Leu Arg Ser Val Arg Asn
                   155                 160                 165
   aac ttc acc ctg ctg acg aac ctt cat gga gcg ccg aac aag agg tca    763
   Asn Phe Thr Leu Leu Thr Asn Leu His Gly Ala Pro Asn Lys Arg Ser
               170                 175                 180
   cca gcg gct agt cag gct cca gtc tcc aga gtc agc ctg caa gag gaa    811
   Pro Ala Ala Ser Gln Ala Pro Val Ser Arg Val Ser Leu Gln Glu Glu
           185                 190                 195
   tca tat cag aaa cta gca atg gag acg ctg gag gaa cta gac tgg tgc    859
   Ser Tyr Gln Lys Leu Ala Met Glu Thr Leu Glu Glu Leu Asp Trp Cys
       200                 205                 210
   cta gac cag cta gag acc atc cag acc tac cgc tct gtc agc gag atg    907
   Leu Asp Gln Leu Glu Thr Ile Gln Thr Tyr Arg Ser Val Ser Glu Met
   215                 220                 225                 230
   gct tca aac aag ttc aaa agg atg ctg aac cgg gag ctg aca cac ctc    955
   Ala Ser Asn Lys Phe Lys Arg Met Leu Asn Arg Glu Leu Thr His Leu
                   235                 240                 245
   tca gag atg agc aga tca ggg aac cag gtg tct gag tac att tca aac   1003
   Ser Glu Met Ser Arg Ser Gly Asn Gln Val Ser Glu Tyr Ile Ser Asn
   250 255 260
   acg ttc tta gac aag cag aac gat gtg gaa atc cca tct ccc acg cag 1051
   Thr Phe Leu Asp Lys Gln Asn Asp Val Glu Ile Pro Ser Pro Thr Gln
   265 270 275
   aag gac agg gag aag aag aag aag cag cag ctc atg acc cag ata agt 1099
   Lys Asp Arg Glu Lys Lys Lys Lys Gln Gln Leu Met Thr Gln Ile Ser
   280 285 290
   gga gtg aag aaa ctg atg cac agc tca agc ctg aac aac aca agc atc 1147
   Gly Val Lys Lys Leu Met His Ser Ser Ser Leu Asn Asn Thr Ser Ile
   295 300 305 310
   tca cgc ttc ggg atc aac acg gaa aat gag gat cat cta gcc aag gag 1195
   Ser Arg Phe Gly Ile Asn Thr Glu Asn Glu Asp His Leu Ala Lys Glu
   315 320 325
   ctg gaa gac ctg aac aaa tgg ggc ctt aac atc ttc aat gtg gct ggg 1243
   Leu Glu Asp Leu Asn Lys Trp Gly Leu Asn Ile Phe Asn Val Ala Gly
   330 335 340
   tac tca cat aat cgg ccc ctt acg tgc atc atg tat gca ata ttc cag 1291
   Tyr Ser His Asn Arg Pro Leu Thr Cys Ile Met Tyr Ala Ile Phe Gln
   345 350 355
   gaa aga gac ctt ctg aag acg ttt aaa atc tca tct gac acc ttt gta 1339
   Glu Arg Asp Leu Leu Lys Thr Phe Lys Ile Ser Ser Asp Thr Phe Val
   360 365 370
   acc tac atg atg act tta gaa gac cat tac cat tct gat gtg gca tat 1387
   Thr Tyr Met Met Thr Leu Glu Asp His Tyr His Ser Asp Val Ala Tyr
   375 380 385 390
   cac aac agc ctg cat gct gct gac gtg gcc cag tca act cac gtt ctc 1435
   His Asn Ser Leu His Ala Ala Asp Val Ala Gln Ser Thr His Val Leu
   395 400 405
   ctt tct acg ccg gca ctg gat gct gtc ttc aca gac ctg gaa atc ctg 1483
   Leu Ser Thr Pro Ala Leu Asp Ala Val Phe Thr Asp Leu Glu Ile Leu
   410 415 420
   gct gcc att ttt gca gct gcc atc cat gat gtc gat cat cct gga gtc 1531
   Ala Ala Ile Phe Ala Ala Ala Ile His Asp Val Asp His Pro Gly Val
   425 430 435
   tcc aat cag ttt ctc atc aat aca aat tct gaa ctt gct ttg atg tat 1579
   Ser Asn Gln Phe Leu Ile Asn Thr Asn Ser Glu Leu Ala Leu Met Tyr
       440                 445                 450
   aat gat gaa tct gtt ctg gaa aac cat cac ctt gct gtg gga ttc aaa    1627
   Asn Asp Glu Ser Val Leu Glu Asn His His Leu Ala Val Gly Phe Lys
   455                 460                 465                 470
   ttg cta caa gag gaa cac tgc gac atc ttt cag aat ctt acc aag aag    1675
   Leu Leu Gln Glu Glu His Cys Asp Ile Phe Gln Asn Leu Thr Lys Lys
                   475                 480                 485
   caa cgc cag aca ctc agg aaa atg gtg att gac atg gtg ttg gca act    1723
   Gln Arg Gln Thr Leu Arg Lys Met Val Ile Asp Met Val Leu Ala Thr
               490                 495                 500
   gat atg tcc aaa cac atg agc ctc ctg gca gac ctt aaa aca atg gta    1771
   Asp Met Ser Lys His Met Ser Leu Leu Ala Asp Leu Lys Thr Met Val
           505                 510                 515
   gaa acc aag aag gtg aca agc tcc ggt gtt ctc ctc ctg gac aac tat    1819
   Glu Thr Lys Lys Val Thr Ser Ser Gly Val Leu Leu Leu Asp Asn Tyr
       520                 525                 530
   act gac cgg ata cag gtt ctt cgc aac atg gta cac tgt gca gac ctg    1867
   Thr Asp Arg Ile Gln Val Leu Arg Asn Met Val His Cys Ala Asp Leu
   535                 540                 545                 550
   agc aac ccc acc aag tcc ttg gaa ttg tat cgg caa tgg acc gat cgt    1915
   Ser Asn Pro Thr Lys Ser Leu Glu Leu Tyr Arg Gln Trp Thr Asp Arg
                   555                 560                 565
   atc atg gag gag ttt ttc cag cag gga gac aaa gaa cgg gag agg gga    1963
   Ile Met Glu Glu Phe Phe Gln Gln Gly Asp Lys Glu Arg Glu Arg Gly
               570                 575                 580
   atg gag att agc cca atg tgt gat aag cac aca gct tct gtg gaa aaa    2011
   Met Glu Ile Ser Pro Met Cys Asp Lys His Thr Ala Ser Val Glu Lys
           585                 590                 595
   tcc cag gtt ggt ttc att gac tac att gtc cat cca ctg tgg gag acc    2059
   Ser Gln Val Gly Phe Ile Asp Tyr Ile Val His Pro Leu Trp Glu Thr
       600                 605                 610
   tgg gca gac ctg gtt caa ccg gat gct caa gat att ctg gat aca cta    2107
   Trp Ala Asp Leu Val Gln Pro Asp Ala Gln Asp Ile Leu Asp Thr Leu
   615                 620                 625                 630
   gaa gat aac agg aac tgg tac cag agt atg ata ccc cag agc cct tcc    2155
   Glu Asp Asn Arg Asn Trp Tyr Gln Ser Met Ile Pro Gln Ser Pro Ser
                   635                 640                 645
   ccg cca ctg gat gag agg agc agg gac tgc caa ggc ctg atg gag aag    2203
   Pro Pro Leu Asp Glu Arg Ser Arg Asp Cys Gln Gly Leu Met Glu Lys
               650                 655                 660
   ttt cag ttt gaa ctg acc ctt gag gaa gag gat tct gag gga ccg gaa    2251
   Phe Gln Phe Glu Leu Thr Leu Glu Glu Glu Asp Ser Glu Gly Pro Glu
           665                 670                 675
   aag gag gga gaa ggc cac agc tat ttc agc agc aca aag acg ctt tgt    2299
   Lys Glu Gly Glu Gly His Ser Tyr Phe Ser Ser Thr Lys Thr Leu Cys
       680                 685                 690
   gtg att gat cca gag aac agg gat tct ctg gaa gag act gac ata gac    2347
   Val Ile Asp Pro Glu Asn Arg Asp Ser Leu Glu Glu Thr Asp Ile Asp
   695                 700                 705                 710
   att gca aca gaa gac aag tct ccg atc gac aca taa tctctctccc         2393
   Ile Ala Thr Glu Asp Lys Ser Pro Ile Asp Thr
                   715                 720
   tctgtgtgga gatgaacatt ccacccttga ctgagcatgc ccgctgagtg gtagggtcac 2453
   ctaccatggc caaggcctgc acaggacaaa ggccacctgg cctttccagt tacttgagtt 2513
   tggagccaga atgccaggcc gtgaagcaaa tagcagttcc atgctgtctt gccttgcctg 2573
   caagcttggc ggagacccgc agctgtatgt ggtagtagag gccagttccc atcaaagcta 2633
   aaatggcttg aaaacagagg acacaaagct gagagattgc tctgcactag gtgttgggaa 2693
   gctgtcctga cagatgactg aactcactaa caacttcatc tataaatctc accacccaac 2753
   ccattgtctg ccaacctgtg tgcctttttt tgtaaaatgt tttcgcgtct ttgaaatgcc 2813
   tgttgaatat ctagagttta gtaccaactt ctacaaactt ttttgagtct ttcttgaaaa 2873
   acaaaaaaaa aaaaaaaaaa aaaaaaaaaa aaaaaaaaa                        2912
<210> 2
   <211> 721
   <212> PRT
   <213> souris
<400> 2
   Met Thr Ala Lys Asn Ser Pro Lys Glu Phe Thr Ala Ser Glu Ser Glu
    1               5                  10                  15
   Val Cys Ile Lys Thr Phe Lys Glu Gln Met Arg Leu Glu Leu Glu Leu
               20                  25                  30
   Pro Lys Leu Pro Gly Asn Arg Pro Thr Ser Pro Lys Ile Ser Pro Arg
           35                  40                  45
   Ser Ser Pro Arg Asn Ser Pro Cys Phe Phe Arg Lys Leu Leu Val Asn
       50                  55                  60
   Lys Ser Ile Arg Gln Arg Arg Arg Phe Thr Val Ala His Thr Cys Phe
   65                  70                  75                  80
   Asp Val Glu Asn Gly Pro Ser Pro Gly Arg Ser Pro Leu Asp Pro Gln
                   85                  90                  95
   Ala Gly Ser Ser Ser Gly Leu Val Leu His Ala Ala Phe Pro Gly His
               100                 105                 110
   Ser Gln Arg Arg Glu Ser Phe Leu Tyr Asp Leu Asp Ser Asp Tyr Asp
           115                 120                 125
   Leu Ser Pro Lys Ala Met Ser Arg Asn Ser Ser Leu Pro Ser Glu Gln
       130                 135                 140
   His Gly Asp Asp Leu Ile Val Thr Pro Phe Ala Gln Val Leu Ala Ser
   145                 150                 155                 160
   Leu Arg Ser Val Arg Asn Asn Phe Thr Leu Leu Thr Asn Leu His Gly
                   165                 170                 175
   Ala Pro Asn Lys Arg Ser Pro Ala Ala Ser Gln Ala Pro Val Ser Arg
               180                 185                 190
   Val Ser Leu Gln Glu Glu Ser Tyr Gln Lys Leu Ala Met Glu Thr Leu
           195                 200                 205
   Glu Glu Leu Asp Trp Cys Leu Asp Gln Leu Glu Thr Ile Gln Thr Tyr
       210                 215                 220
   Arg Ser Val Ser Glu Met Ala Ser Asn Lys Phe Lys Arg Met Leu Asn
   225                 230                 235                 240
   Arg Glu Leu Thr His Leu Ser Glu Met Ser Arg Ser Gly Asn Gln Val
                   245                 250                 255
   Ser Glu Tyr Ile Ser Asn Thr Phe Leu Asp Lys Gln Asn Asp Val Glu
               260                 265                 270
   Ile Pro Ser Pro Thr Gln Lys Asp Arg Glu Lys Lys Lys Lys Gln Gln
           275                 280                 285
   Leu Met Thr Gln Ile Ser Gly Val Lys Lys Leu Met His Ser Ser Ser
       290                 295                 300
   Leu Asn Asn Thr Ser Ile Ser Arg Phe Gly Ile Asn Thr Glu Asn Glu
   305                 310                 315                 320
   Asp His Leu Ala Lys Glu Leu Glu Asp Leu Asn Lys Trp Gly Leu Asn
                   325                 330                 335
   Ile Phe Asn Val Ala Gly Tyr Ser His Asn Arg Pro Leu Thr Cys Ile
               340                 345                 350
   Met Tyr Ala Ile Phe Gln Glu Arg Asp Leu Leu Lys Thr Phe Lys Ile
           355                 360                 365
   Ser Ser Asp Thr Phe Val Thr Tyr Met Met Thr Leu Glu Asp His Tyr
       370                 375                 380
   His Ser Asp Val Ala Tyr His Asn Ser Leu His Ala Ala Asp Val Ala
   385 390 395 400
   Gln Ser Thr His Val Leu Leu Ser Thr Pro Ala Leu Asp Ala Val Phe
   405 410 415
   Thr Asp Leu Glu Ile Leu Ala Ala Ile Phe Ala Ala Ala Ile His Asp
   420 425 430
   Val Asp His Pro Gly Val Ser Asn Gln Phe Leu Ile Asn Thr Asn Ser
   435 440 445
   Glu Leu Ala Leu Met Tyr Asn Asp Glu Ser Val Leu Glu Asn His His
   450 455 460
   Leu Ala Val Gly Phe Lys Leu Leu Gln Glu Glu His Cys Asp Ile Phe
   465 470 475 480
   Gln Asn Leu Thr Lys Lys Gln Arg Gln Thr Leu Arg Lys Met Val Ile
   485 490 495
   Asp Met Val Leu Ala Thr Asp Met Ser Lys His Met Ser Leu Leu Ala
   500 505 510
   Asp Leu Lys Thr Met Val Glu Thr Lys Lys Val Thr Ser Ser Gly Val
   515 520 525
   Leu Leu Leu Asp Asn Tyr Thr Asp Arg Ile Gln Val Leu Arg Asn Met
   530 535 540
   Val His Cys Ala Asp Leu Ser Asn Pro Thr Lys Ser Leu Glu Leu Tyr
   545 550 555 560
   Arg Gln Trp Thr Asp Arg Ile Met Glu Glu Phe Phe Gln Gln Gly Asp
   565 570 575
   Lys Glu Arg Glu Arg Gly Met Glu Ile Ser Pro Met Cys Asp Lys His
   580 585 590
   Thr Ala Ser Val Glu Lys Ser Gln Val Gly Phe Ile Asp Tyr Ile Val
   595 600 605
   His Pro Leu Trp Glu Thr Trp Ala Asp Leu Val Gln Pro Asp Ala Gln
   610 615 620
   Asp Ile Leu Asp Thr Leu Glu Asp Asn Arg Asn Trp Tyr Gln Ser Met
   625 630 635 640
   Ile Pro Gln Ser Pro Ser Pro Pro Leu Asp Glu Arg Ser Arg Asp Cys
   645 650 655
   Gln Gly Leu Met Glu Lys Phe Gln Phe Glu Leu Thr Leu Glu Glu Glu
   660 665 670
   Asp Ser Glu Gly Pro Glu Lys Glu Gly Glu Gly His Ser Tyr Phe Ser
   675 680 685
   Ser Thr Lys Thr Leu Cys Val Ile Asp Pro Glu Asn Arg Asp Ser Leu
   690 695 700
   Glu Glu Thr Asp Ile Asp Ile Ala Thr Glu Asp Lys Ser Pro Ile Asp
   705 710 715 720
   Thr
<210> 3
   <211> 4068
   <212> ADN
   <213> Homo sapiens
<220>
   <221> CDS
   <222> (766)..(2460)
   <223> PDE4B
<400> 3
<210> 4
   <211> 564
   <212> PRT
   <213> Homo sapiens
<400> 4
   Met Lys Glu His Gly Gly Thr Phe Ser Ser Thr Gly Ile Ser Gly Gly
    1               5                  10                  15
   Ser Gly Asp Ser Ala Met Asp Ser Leu Gln Pro Leu Gln Pro Asn Tyr
               20                  25                  30
   Met Pro Val Cys Leu Phe Ala Glu Glu Ser Tyr Gln Lys Leu Ala Met
           35                  40                  45
   Glu Thr Leu Glu Glu Leu Asp Trp Cys Leu Asp Gln Leu Glu Thr Ile
       50                  55                  60
   Gln Thr Tyr Arg Ser Val Ser Glu Met Ala Ser Asn Lys Phe Lys Arg
   65                  70                  75                  80
   Met Leu Asn Arg Glu Leu Thr His Leu Ser Glu Met Ser Arg Ser Gly
                   85                  90                  95
   Asn Gln Val Ser Glu Tyr Ile Ser Asn Thr Phe Leu Asp Lys Gln Asn
               100                 105                 110
   Asp Val Glu Ile Pro Ser Pro Thr Gln Lys Asp Arg Glu Lys Lys Lys
           115                 120                 125
   Lys Gln Gln Leu Met Thr Gln Ile Ser Gly Val Lys Lys Leu Met His
       130                 135                 140
   Ser Ser Ser Leu Asn Asn Thr Ser Ile Ser Arg Phe Gly Val Asn Thr
   145                 150                 155                 160
   Glu Asn Glu Asp His Leu Ala Lys Glu Leu Glu Asp Leu Asn Lys Trp
                   165                 170                 175
   Gly Leu Asn Ile Phe Asn Val Ala Gly Tyr Ser His Asn Arg Pro Leu
               180                 185                 190
   Thr Cys Ile Met Tyr Ala Ile Phe Gln Glu Arg Asp Leu Leu Lys Thr
           195                 200                 205
   Phe Arg Ile Ser Ser Asp Thr Phe Ile Thr Tyr Met Met Thr Leu Glu
       210                 215                 220
   Asp His Tyr His Ser Asp Val Ala Tyr His Asn Ser Leu His Ala Ala
   225                 230                 235                 240
   Asp Val Ala Gln Ser Thr His Val Leu Leu Ser Thr Pro Ala Leu Asp
                   245                 250                 255
   Ala Val Phe Thr Asp Leu Glu Ile Leu Ala Ala Ile Phe Ala Ala Ala
               260                 265                 270
   Ile His Asp Val Asp His Pro Gly Val Ser Asn Gln Phe Leu Ile Asn
           275                 280                 285
   Thr Asn Ser Glu Leu Ala Leu Met Tyr Asn Asp Glu Ser Val Leu Glu
       290                 295                 300
   Asn His His Leu Ala Val Gly Phe Lys Leu Leu Gln Glu Glu His Cys
   305                 310                 315                 320
   Asp Ile Phe Met Asn Leu Thr Lys Lys Gln Arg Gln Thr Leu Arg Lys
                   325                 330                 335
   Met Val Ile Asp Met Val Leu Ala Thr Asp Met Ser Lys His Met Ser
               340                 345                 350
   Leu Leu Ala Asp Leu Lys Thr Met Val Glu Thr Lys Lys Val Thr Ser
           355                 360                 365
   Ser Gly Val Leu Leu Leu Asp Asn Tyr Thr Asp Arg Ile Gln Val Leu
       370                 375                 380
   Arg Asn Met Val His Cys Ala Asp Leu Ser Asn Pro Thr Lys Ser Leu
   385 390 395 400
   Glu Leu Tyr Arg Gln Trp Thr Asp Arg Ile Met Glu Glu Phe Phe Gln
   405 410 415
   Gln Gly Asp Lys Glu Arg Glu Arg Gly Met Glu Ile Ser Pro Met Cys
   420 425 430
   Asp Lys His Thr Ala Ser Val Glu Lys Ser Gln Val Gly Phe Ile Asp
   435 440 445
   Tyr Ile Val His Pro Leu Trp Glu Thr Trp Ala Asp Leu Val Gln Pro
   450 455 460
   Asp Ala Gln Asp Ile Leu Asp Thr Leu Glu Asp Asn Arg Asn Trp Tyr
   465 470 475 480
   Gln Ser Met Ile Pro Gln Ser Pro Ser Pro Pro Leu Asp Glu Gln Asn
   485 490 495
   Arg Asp Cys Gln Gly Leu Met Glu Lys Phe Gln Phe Glu Leu Thr Leu
   500 505 510
   Asp Glu Glu Asp Ser Glu Gly Pro Glu Lys Glu Gly Glu Gly His Ser
   515 520 525
   Tyr Phe Ser Ser Thr Lys Thr Leu Cys Val Ile Asp Pro Glu Asn Arg
   530 535 540
   Asp Ser Leu Gly Glu Thr Asp Ile Asp Ile Ala Thr Glu Asp Lys Ser
   545 550 555 560
   Pro Val Asp Thr
<210> 5
   <211> 20
   <212> ADN
   <213> Séquence artificielle
<220>
   <223> Description de la séquence artificielle: amorce
<400> 5
   gccaggccgt gaagcaaata 20
<210> 6
   <211> 18
   <212> ADN
   <213> Séquence artificielle
<220>
   <223> Description de la séquence artificielle: amorce
<400> 6
   tcaaagacgc gaaaacat 18
<210> 7
   <211> 21
   <212> ADN
   <213> Séquence artificielle
<220>
   <223> Description de la séquence artificielle: amorce
<400> 7
   ccgcgtcagt gcctttgcta t 21
<210> 8
   <211> 23
   <212> ADN
   <213> Séquence artificielle
<220>
   <223> Description de la séquence artificielle: amorce
<400> 8
   cgctgtcgga tgcttttatt cac 23
<210> 9
   <211> 19
   <212> ADN
   <213> Séquence artificielle
<220>
   <223> Description de la séquence artificielle: amorce
<400> 9
   tcgctttctg gagggtgtc 19
<210> 10
   <211> 18
   <212> ADN
   <213> Séquence artificielle
<220>
   <223> Description de la séquence artificielle: amorce
<400> 10
   ccgcaggggc agcagtgg 18